(19) Europäisches Patentamt / European Patent Office / Office européen des brevets

(11) **EP 4 582 539 A1**

(12) **EUROPEAN PATENT APPLICATION**
published in accordance with Art. 153(4) EPC

(43) Date of publication:
09.07.2025 Bulletin 2025/28

(21) Application number: 23859747.0

(22) Date of filing: 26.05.2023

(51) International Patent Classification (IPC):
$C12N\ 15/09^{(2006.01)}$     $C12Q\ 1/68^{(2018.01)}$
$C12Q\ 1/6827^{(2018.01)}$     $C12Q\ 1/6851^{(2018.01)}$
$C12Q\ 1/6869^{(2018.01)}$

(52) Cooperative Patent Classification (CPC):
C12N 15/09; C12Q 1/68; C12Q 1/6827;
C12Q 1/6851; C12Q 1/6869

(86) International application number:
PCT/JP2023/019597

(87) International publication number:
WO 2024/047977 (07.03.2024 Gazette 2024/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.08.2022 JP 2022137059
22.12.2022 JP 2022205502

(71) Applicant: Seedna Inc.
Tokyo 121-0813 (JP)

(72) Inventor: TOMIKANE, Kinori
Tokyo 121-0813 (JP)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) **GENETIC ANALYSIS METHOD WHEREBY TWO OR MORE TYPES OF TESTS CAN BE PERFORMED**

(57) The present invention addresses the problem of providing novel technology whereby it is possible to perform two or more tests selected from among parentage testing, phenotype analysis, and diagnosis of chromosomal numerical abnormalities. The present invention is a genetic analysis method for analyzing a group , which is satisfying two or more conditions selected from (i) to (iii) and includes two or more steps selected from a phenotype analysis step, a Parentage Testing step, and an Calculation Step for Evaluating Chromosomal Numerical Abnormalities.(i) The proportion of the number of single nucleotide polymorphism loci, which is already known to have a phenotype among the total number of single nucleotide polymorphism loci included in the group to be analyzed is 10% or more. (ii) The proportion of the number of single nucleotide polymorphism loci that have a minor allele frequency of 1%-50% and are separated from each other by a distance of 20 kb or more on the chromosome among the total number of single nucleotide polymorphism loci included in the group to be analyzed is 10% or more. (iii) The proportion of the number of single nucleotide polymorphism loci present on chromosome 13, chromosome 18, chromosome 21, the X chromosome, and the Y chromosome among the total number of single nucleotide polymorphism loci included in the group to be analyzed is 10% or more.

Fig.1

EP 4 582 539 A1

## Description

Field of the Invention

The present invention relates to the technical field of nucleic acid analysis methods using next-generation sequencers and similar technologies.

Background Art

Advancements in human genome and genetic analysis research have enabled the identification of causative genes for many monogenic diseases and have also facilitated the rapid elucidation of genetic factors associated with multifactorial diseases. Genome-wide association study (GWAS) is an effective approach for identifying genetic variants. This method enables the identification of genes associated with disease risk and quantitative traits in humans, such as body mass index (BMI) and blood metabolite concentrations. The association between a mutation identified by GWAS and a phenotype is expressed by a P value.

With the development of such technologies, the implementation of personalized medicine, which provides optimal medical care based on an individual's genetic background, is being realized. With such technological innovation, genetic analysis is becoming increasingly accessible to consumers. Direct-to-consumer genetic testing (hereinafter referred to as "DTC genetic testing") provides information on disease risk, genetic predisposition, and personality that encourages consumers to improve their behavior. Furthermore, it is also extended to include secondary services such as the provision of diet programs and the sale of cosmetics and supplements (see, for example, Non-patent document 1).

Furthermore, uncertainty about a parent-child relationship has a significant impact on legal and family matters. If there is uncertainty regarding the biological paternity of a fetus during pregnancy, several methods exist to determine the correct biological father.

One method is to wait until birth, analyze the genomic DNA of the child and the alleged father, and perform STR analysis using Fragment analysis, which is used in existing paternity tests.

However, there is a high demand for knowing the biological father of a child before birth. Methods for discriminating parent-child relationships before birth include analyzing genetic material collected by chorionic villus sampling or amniocentesis, but these are invasive and have the problem of miscarriage risk.

In view of the problems of the invasive prenatal diagnostic methods described above, methods of analyzing cell-free DNA (cfDNA) mixed in blood have been applied to paternity testing. By analyzing circulating fetal cell-free DNA (cffDNA), which is fetal genetic material mixed in the mother's blood circulation, it becomes possible to perform Non-Invasive Prenatal Paternity Testing (NIPPT).

In this case, since it is difficult to analyze fragmented cffDNA with existing STR analysis, the MPS (massive parallel sequencing) method with multiple SNV Genotyping is used.

For example, Patent Document 1 discloses a method of NIPPT in which the probability that the alleged father is the biological father of the fetus is determined by a computer using a maximum likelihood estimate or a maximum a posteriori probability method, and based on the determined probability, a single hypothesis rejection test, maximum likelihood estimation, or maximum a posteriori probability method is used to confirm whether or not the alleged father is the biological father of the fetus.

Non-Patent Document 2 discloses a method utilizing a Bayesian-based algorithm in maternal-fetal genotype prediction.

Non-Patent Document 3 describes that the number of effective SNVs has a significant relationship with the minor allele frequency (MAF) and the total number of SNVs, and that the sequencing depth affects the accuracy of NIPPT.

Another application of fetal cell-free DNA analysis technology is the detection of fetal chromosomal aneuploidies by Non-Invasive Prenatal Genetic Testing (NIPT). Chromosomal aneuploidies are numerical abnormalities of chromosomes, including trisomy 21 (Down syndrome), trisomy 18 (Edwards syndrome), trisomy 13 (Patau syndrome), and monosomy X (Turner syndrome).

A known method for NIPT based on fetal cell-free DNA is called the quantitative DNA comparison method or counting method (Massively-Parallel Sequencing method, MPS method).

In NIPT, the count or signal intensity is analyzed, and the deviation from the normal reference (median) is quantified and evaluated.

Prior Art Documents

Patent Documents

Patent Document 1: Japanese Patent Application National Publication No. 2014-502845
Patent Document 2: Japanese Patent Application National Publication No. 2022-519159
Patent Document 3: Japanese Unexamined Patent Application Publication No. 2019-153332

Non-Patent Documents

Non-Patent Document 1: Nutrients. 2020 Feb; 12(2): 566. Published online 2020 Feb 21.
Non-Patent Document 2: Prenatal Diagnosis, Volume 40, Issue 4, March 2020, Pages 497-506.
Non-Patent Document 3: PLoS One. 2016 Sep 15; 11(9): e0159385.
Non-Patent Document 4: PLoS One. 2019 Sep 12; 14(9): e0222535.
Non-Patent Document 5: PLoS One. 2019 Apr 12; 14(4): e0215368.
Non-Patent Document 6: Clin. Chem. 2011 Jul; 57(7): 1042-9.

Summary of Invention

Problems to be Solved by the Invention

DTC genetic testing, phenotype analysis such as single-gene disorder testing, parentage testing, and NIPT have been recognized as separate technologies and services, and the concept of analyzing them simultaneously in a single test had not been considered. Because of this lack of consideration, no effort had been made to determine how to construct a panel for simultaneously performing these tests.
An object of the present invention is to provide a novel technique that enables the performance of two or more tests selected from parentage testing, phenotype analysis, and diagnosing chromosomal aneuploidy.

Means for Solving the Problems

The present invention provides the following.
[1] A method for genetic analysis of a set of targets of analysis comprising multiple single nucleotide polymorphism loci, wherein:

the set of targets of analysis meets at least two of the following conditions (i) to (iii):

(i) The ratio of the number of single nucleotide polymorphism loci, which is known to have a phenotype to the total number of single nucleotide polymorphism loci in the set of targets of analysis is 10% or more;

(ii) The ratio of the number of single nucleotide polymorphism loci, among the total number of single nucleotide polymorphism loci in the set of targets of analysis, where the minor allele frequency is 1% to 50% and which are separated from each other by a distance of 20 kb or more on a chromosome, is 10% or more;

(iii) The ratio of the number of single nucleotide polymorphism loci present on chromosome 13, chromosome 18, chromosome 21, chromosome X, and chromosome Y to the total number of single nucleotide polymorphism loci in the set of targets of analysis is 10% or more;

comprising a genotyping step, wherein:

the genotyping step includes analyzing a child's nucleic acid sample containing a nucleic acid with the genetic information of a child, and a father's nucleic acid sample containing a nucleic acid with the genetic information of a father and/or a mother's nucleic acid sample containing a nucleic acid with the genetic information of a mother,
to acquire data including:

- allele sequence information , which is multiple single nucleotide polymorphism loci in the set of targets of analysis in the nucleic acids of each sample; and

- numerical values reflecting the genomic region containing these loci and/or the relative or absolute abundance of the alleles. ;

further comprising executing at least two steps selected from the following: a Phenotype Analysis Step, a Parentage Testing Step, and a Calculation Step for Evaluating Chromosomal Numerical Abnormalities, based on the data obtained from a single analysis,
wherein when the Phenotype Analysis Step is included, the set of targets of analysis meets condition (i),
when the Parentage Testing Step is included, the set of targets of analysis meets condition (ii), and
when the Calculation Step for Evaluating Chromosomal Numerical Abnormalities is included, the set of targets of analysis meets condition (iii).

<Step for Phenotype Analysis>

A step of analyzing, based on the genotyping results of single nucleotide polymorphism loci, which is known to be associated with the phenotype, obtained in the genotyping step, the likelihood of expression of the phenotype associated with the minor allele at the single nucleotide polymorphism loci, in at least the child sample,
excluding the diagnosis of a disease in a human.

<Parentage Testing Step>

A step of verifying the existence of a parent-child relationship, comprising:

- determining a Paternity Index (PI) for each of the plurality of single nucleotide polymorphism loci based on the result of the genotyping step and multiplying the PIs to obtain a Combined Paternity Index (CPI), or
- determining a Maternity Index (MI) for each of the plurality of single nucleotide polymorphism loci based on the result of the genotyping step and multiplying the MIs to obtain a Combined Maternity Index (CMI),

wherein PI or MI is calculated as follows for the following cases:

- the child is a fetus before birth and paternity testing is performed,
- the child is after birth and paternity testing is performed, or
- the child is after birth and maternity testing is performed.

Case where the child is a fetus before birth and paternity testing is performed:
The child's nucleic acid sample is a cell-free nucleic acid sample collected from the pregnant mother with the child.

- When genotyping of the father and the fetus is performed by analyzing two types of samples, the cell-free nucleic acid sample and the father's nucleic acid sample, PI is determined based on Table 1 or Table 2.
- When genotyping of the father, the mother, and the fetus is performed by analyzing three types of samples, the cell-free nucleic acid sample, the father's nucleic acid sample, and the mother's nucleic acid sample, PI is determined based on Table 3 or Table 4.

Case where the child is after birth and paternity testing is performed:

- When genotyping of the father and the child is performed by analyzing two types of samples, the child's nucleic acid sample and the father's nucleic acid sample, PI is determined based on Table 5.
- When genotyping of the father, the mother, and the child is performed by analyzing three types of samples, the child's nucleic acid sample, the father's nucleic acid sample, and the mother's nucleic acid sample, PI is determined based on Table 6.

Case where the child is after birth and maternity testing is performed:

- When genotyping of the mother and the child is performed by analyzing two types of samples, the child's nucleic acid sample and the mother's nucleic acid sample, MI is determined based on Table 7.
- When genotyping of the father, the mother, and the child is performed by analyzing three types of samples, the child's nucleic acid sample, the father's nucleic acid sample, and the mother's nucleic acid sample, MI is determined based on Table 8.

Table 1

| CASE | Genotype | | Paternity Index |
|---|---|---|---|
| | alleged father | fetus | PI |
| (I) | AA | AA | $\dfrac{1-k_0}{(1-k_0)a+k_0 b}$ |
| (II) | AA | AB | $\dfrac{k_b}{k_b a+(1-k_b)b}$ |
| (III) | BB | AA | $\dfrac{k_0}{(1-k_0)a+k_0 b}$ |
| (IV) | BB | AB | $\dfrac{1-k_b}{k_b a+(1-k_b)b}$ |
| (V) | AB | AA | $\dfrac{1}{2(1-k_0)a+2k_0 b}$ |
| (VI) | AB | AB | $\dfrac{1}{2k_b a+2(1-k_b)b}$ |

Table 2

| CASE | Genotype | | Paternity Index |
|---|---|---|---|
| | alleged father | fetus | PI |
| (I) | AA | AA | 1/a |
| (II) | AA | AB | kb |
| (III) | BB | AA | k0 |
| (IV) | BB | AB | 1/b |
| (V) | AB | AA | 0.5/a |
| (VI) | AB | AB | 0.5/b |

Table 3

| CASE | Genotype | | | Paternity Index |
|---|---|---|---|---|
| | mother | alleged father | fetus | PI |
| (I) | AA | AA | AA | $\dfrac{1-k_0}{(1-k_0)a+k_0 b}$ |
| (II) | AA | AA | AB | $\dfrac{k_b}{k_b a+(1-k_b)b}$ |
| (III) | AA | BB | AA | $\dfrac{k_0}{(1-k_0)a+k_0 b}$ |
| (IV) | AA | BB | AB | $\dfrac{1-k_b}{k_b a+(1-k_b)b}$ |

(continued)

| CASE | Genotype | | | Paternity Index |
|---|---|---|---|---|
| | mother | alleged father | fetus | PI |
| (V) | AA | AB | AA | $\dfrac{1}{2(1-k_0)a + 2k_0 b}$ |
| (VI) | AA | AB | AB | $\dfrac{1}{2k_b a + 2(1-k_b)b}$ |

Table 4

| CASE | Genotype | | Paternity Index | |
|---|---|---|---|---|
| | mother | alleged father | fetus | PI |
| (I) | AA | AA | AA | 1/a |
| (II) | AA | AA | AB | kb |
| (III) | AA | BB | AA | k0 |
| (IV) | AA | BB | AB | 1/b |
| (V) | AA | AB | AA | 0.5/a |
| (VI) | AA | AB | AB | 0.5/b |

Table 5

| CASE | Genotype | | Paternity Index |
|---|---|---|---|
| | alleged father | child | PI |
| (I) | AA | AA | 1/a |
| (II) | AA | AB | 0.5/a |
| (III) | BB | AA | $\mu/\beta$ |
| (IV) | AB | AA | 0.5/a |
| (V) | AB | AB | (a+b) 4ab |

Table 6

| CASE | Genotype | | | Paternity Index |
|---|---|---|---|---|
| | mother | alleged father | child | PI |
| (I) | AA | AA | AA | 1/a |
| (II) | AA | AA | AB | $\mu/\beta$ |
| (III) | AA | BB | AA | $\mu/\alpha$ |
| (IV) | AA | BB | AB | 1/b |
| (V) | AA | AB | AA | 0.5/a |
| (VI) | AA | AB | AB | 1/ (a+b) |

Table 7

| CASE | Genotype | | Paternity Index |
|---|---|---|---|
| | alleged mather | child | PI |
| (I) | AA | AA | 1/a |

(continued)

| CASE | Genotype | | Paternity Index |
|------|----------|-------|------|
| | alleged mather | child | PI |
| (II) | AA | AB | 0.5/a |
| (III) | BB | AA | μ/β |
| (IV) | AB | AA | 0.5/a |
| (V) | AB | AB | (a+b) 4ab |

Table 8

| CASE | Genotype | | | Paternity Index |
|------|----------|--------|-------|------|
| | alleged mother | father | child | PI |
| (I) | AA | AA | AA | 1/a |
| (II) | AA | AA | AB | μ/β |
| (III) | AA | BB | AA | μ/α |
| (IV) | AA | BB | AB | 1/b |
| (V) | AA | AB | AA | 0.5/a |
| (VI) | AA | AB | AB | 1/ (a+b) |

In Tables 1 to 4, the allele with relatively high signal intensity in the analysis of the cell-free nucleic acid sample is referred to as A, and the allele with relatively low signal intensity is referred to as B.

In Tables 1 and 2, k0 is a false negative rate, kb is a false positive rate, a is the frequency of A, and b is the frequency of B.

In Tables 5 to 8, μ is the mutation rate, a and b are the occurrence frequencies of allele A and allele B, respectively, α is the average exclusion power of allele A, and β is the average exclusion power of allele B.

<Calculation Step for Evaluating Chromosomal Numerical Abnormalities>

A step of calculating a Z score or NCV (Normalized Chromosome Value) as an index to evaluate the copy number variation of chromosome 13, chromosome 18, chromosome 21, chromosome X, and chromosome Y in at least the child, based on the numerical values contained in the data,
wherein the Z score (Z chrN) for chromosome N is calculated according to the following equation (1), and the NCV (NCV chrN by chrM) for chromosome N relative to reference chromosome M is calculated according to the following equation (2).

$$Z_{chrN} = \frac{\text{proportion of chr N} - \text{mean proportion of chr N in reference disomic population}}{\text{SD of proportion of chr N in reference disomic population}}$$

…equation (1)

(In equation (1), N is 13, 18, 21, X, or Y, "proportion of chr N" is the ratio of the numerical value reflecting the abundance of chromosome N to the total numerical value reflecting the abundance of all chromosomes except chromosomes 13, 18, 21, X, and Y, when analyzing the child's nucleic acid sample.

"mean of proportion of chr N in reference disomic population" is the mean ratio of the numerical value reflecting the abundance of chromosome N to the total numerical value reflecting the abundance of all chromosomes except chromosomes 13, 18, 21, X, and Y, when analyzing multiple standard samples with a normal disomic karyotype.

"SD of proportion of chr N in reference disomic population" is the standard deviation of the ratio of the numerical value reflecting the abundance of chromosome N to the total numerical value reflecting the abundance of all chromosomes except chromosomes 13, 18, 21, X, and Y, when analyzing multiple standard samples with a normal disomic karyotype.)

$$NCV_{chr\,N\,by\,chr\,M}$$

$$= \frac{Normalized\ proportion\ of\ chr\ N\ by\ chr\ M - mean\ normalized\ proportion\ of\ chr\ N\ in\ reference\ disomic\ population}{SD\ of\ normalized\ proportion\ of\ chr\ N\ in\ reference\ disomic\ population}$$

...equation (2)

In equation (2),
"Normalized proportion of chr N by chr M" is the ratio of the numerical value reflecting the abundance of chromosome N relative to the numerical value reflecting the abundance of chromosome M in the child's nucleic acid sample.

"mean normalized proportion of chr N in reference disomic population" is the mean ratio of the numerical value reflecting the abundance of chromosome N relative to the numerical value reflecting the abundance of chromosome M when analyzing multiple standard samples with a normal disomic karyotype.

"SD of normalized proportion of chr N in reference disomic population" is the standard deviation of the ratio of the numerical value reflecting the abundance of chromosome N relative to the numerical value reflecting the abundance of chromosome M when analyzing multiple standard samples with a normal disomic karyotype.

[2] The genetic analysis method according to item 1, comprising all of the Parentage Testing Step, the Calculation Step for Evaluating Chromosomal Numerical Abnormalities, and the Phenotype Analysis Step.

[3] The genetic analysis method according to item 1 or 2, wherein the set of targets of analysis satisfies all the conditions set forth in (i) to (iii), and the ratio of the number of single nucleotide polymorphism loci that are known to have a phenotype, have a minor allele frequency of 1% to 50%, are separated from each other by a distance of 20 kb or more on a chromosome, and present on chromosome 13, chromosome 18, chromosome 21, chromosome X, and chromosome Y, to the total number of single nucleotide polymorphism loci constituting the set of targets of analysis is 10% or more.

[4] The genetic analysis method according to any one of items 1 to 3, wherein the child is a fetus in the mother's womb, and the child's nucleic acid sample is a cell-free nucleic acid sample collected from the mother.

[5] The genetic analysis method according to any one of items 1 to 3, wherein the child is postnatal.

Effects of the Invention

By analyzing single nucleotide polymorphism loci of a set of targets of analysis that satisfies two or more conditions selected from the above (i) to (iii), it becomes possible to perform two or more genetic tests selected from Parentage Testing Step, Calculation Step for Evaluating Chromosomal Numerical Abnormalities, and Phenotype Analysis Step. The technical effects will be described in more detail below.

<Technical Effect 1> Combination of Parentage Testing Step and Phenotype Analysis Step When the Child is a Fetus

When the Parentage Testing Step and the Phenotype Analysis Step are combined, by confirming the parent-child relationship between the fetus and the father in the Parentage Testing Step, it becomes possible to determine whether the minor allele inherited by the fetus originates from the father or the mother in the Phenotype Analysis Step. This makes it possible to more accurately estimate the likelihood of expression of a phenotype related to the minor allele in the fetus.

Further, when the parent-child relationship between the fetus and the father can be confirmed in the Parentage Testing Step, it is possible to refine the probability estimation that the fetus actually possesses the minor allele whose presence is suggested by genotyping.

Specifically,

1) when one parent possesses the minor allele as a homozygote, it can be determined that the fetus possesses the minor allele with certainty,
2) when one parent possesses the minor allele as a heterozygote, it can be determined that the likelihood that the fetus actually possesses the minor allele is higher, and
3) when both parents possess the minor allele as heterozygotes, it can be determined that the likelihood that the fetus actually possesses the minor allele is higher than in the above 2).

These technical effects cannot be obtained when the Parentage Testing Step and the Phenotype Analysis Step are performed separately, and are effects that are achieved for the first time by combining these steps.

<Technical Effect 2> Combination of Calculation Step for Evaluating Chromosomal Numerical Abnormalities and Phenotype Analysis Step When the Child is a Fetus

When only the Phenotype Analysis Step is performed, it only allows determining whether the fetus has a minor allele. However, by combining the Phenotype Analysis Step and the Calculation Step for Evaluating Chromosomal Numerical Abnormalities, if the minor allele corresponds to a loss-of-function mutation or a dominant-negative mutation, it becomes possible to assess the potential severity of the disease by identifying chromosomal numerical abnormalities.
This technical effect cannot be obtained when the Calculation Step for Evaluating Chromosomal Numerical Abnormalities and the Phenotype Analysis Step are performed separately, and is an effect that is achieved for the first time by combining these steps.

<Technical Effect 3> Combination of Parentage Testing Step and Calculation Step for Evaluating Chromosomal Numerical Abnormalities When the Child is a Fetus

Since the cell-free nucleic acid sample obtained from the pregnant mother contains a mixture of maternal nucleic acid and fetal nucleic acid, there is a risk that the signal indicating a specific allele obtained by analysis may be misattributed to either the mother or the fetus, leading to an erroneous conclusion in the evaluation of chromosomal numerical abnormalities.
However, by confirming the parent-child relationship between the father and the fetus in the Parentage Testing Step, it becomes possible to determine whether the signal indicating a specific allele obtained by analyzing the cell-free nucleic acid sample originates from the fetus (that is, whether it was inherited from the father through meiosis), thereby enabling the accurate evaluation of the fetal nucleic acid content. This can improve the accuracy of chromosomal numerical abnormality evaluation.
This technical effect cannot be obtained when the Parentage Testing Step and the Calculation Step for Evaluating Chromosomal Numerical Abnormalities are performed separately, and is an effect that is achieved for the first time by combining these steps.

<Technical Effect 4> Combination of Parentage Testing Step and Phenotype Analysis Step When the Child is Postnatal

When the Parentage Testing Step and the Phenotype Analysis Step are combined, by confirming the parent-child relationship between the child and the father in the Parentage Testing Step, it becomes possible to determine whether the minor allele inherited by the child originates from the father or the mother in the Phenotype Analysis Step.
This technical effect cannot be obtained when the Parentage Testing Step and the Phenotype Analysis Step are performed separately, and is an effect that is achieved for the first time by combining these steps.

<Technical Effect 5> Combination of Calculation Step for Evaluating Chromosomal Numerical Abnormalities and Phenotype Analysis Step When the Child is Postnatal

By combining the Phenotype Analysis Step and the Calculation Step for Evaluating Chromosomal Numerical Abnormalities, if the minor allele corresponds to a loss-of-function mutation or a dominant-negative mutation, it becomes possible to assess the potential severity of the disease by identifying chromosomal numerical abnormalities.
This technical effect cannot be obtained when the Calculation Step for Evaluating Chromosomal Numerical Abnormalities and the Phenotype Analysis Step are performed separately, and is an effect that is achieved for the first time by combining these steps.

<Technical Effect 6> Combination of Parentage Testing Step and Calculation Step for Evaluating Chromosomal Numerical Abnormalities When the Child is Postnatal

Chromosomal numerical abnormalities are caused by errors in meiosis during the production of sperm or eggs. If a numerical abnormality is found in a specific chromosome in a child, it indicates that there was an error in meiosis in either the sperm or the egg. When simply evaluating the chromosomal numerical abnormality of the child, it is not possible to determine whether the cause of the numerical abnormality is due to an error in meiosis of the sperm (father) or the egg (mother).
However, in the present invention, since "data including allele sequence information at a plurality of single nucleotide polymorphism loci included in the set of targets of analysis, and numerical values reflecting the region including the single nucleotide polymorphism loci and/or the relative or absolute abundance of the alleles is acquired", it is possible to detect quantitative abnormalities by distinguishing the difference in alleles at a specific single nucleotide polymorphism locus. If the biological parent-child relationship between the child and the father or mother can be confirmed by the Parentage Testing Step, it is possible to determine whether the allele at the single nucleotide polymorphism locus originates from the father or the mother. Then, it is possible to determine whether the specific allele with an abnormal ploidy originates from the father or the mother, thereby identifying whether the cause of the chromosomal numerical abnormality is due to an error in meiosis of the sperm (father) or the egg (mother).

This technical effect cannot be obtained when the Parentage Testing Step and the calculation step for evaluating chromosomal numerical abnormalities are performed separately, and is an effect that is achieved for the first time by combining these steps.

Brief Description of the Drawings

[Figure 1] A graph showing the simulation results of Test Example 1. As the allele frequency a of the minor allele increases, the value of CPI increases in proportion to the number of SNVs.
[Figure 2] A graph showing the simulation results of Test Example 2. As the allele frequency a of the minor allele increases, the value of CPI increases in proportion to the number of SNVs.

Mode for Carrying Out the Invention

Embodiments of the present invention will be described below. In "<1> Genotyping for Single Nucleotide Polymorphism Loci of the Set of Targets of Analysis ", the panel used in the present invention and genotyping using the panel will be described. In the items "<2> Prenatal" and "<3> Postnatal", embodiments of tests for children before and after birth will be described, respectively.

<1> Genotyping for Single Nucleotide Polymorphism Loci of the Set of Targets of Analysis

Conventionally, the SNV panel used for parentage testing is composed of SNV loci capable of individual identification, and the relationship with phenotype and the detection of chromosomal numerical abnormalities are not considered in the construction of the panel. Therefore, analysis using the SNV panel used for parentage testing cannot simultaneously perform DTC genetic testing or single gene disorder testing for the purpose of predicting phenotypes such as the possibility of disease susceptibility and constitution of a subject, and evaluation of numerical abnormalities such as chromosome 13, chromosome 18, chromosome 21, chromosome X, and chromosome Y (Patent Document 1, Non-Patent Documents 2, 3).

Conventionally, when performing DTC genetic testing or the like, a panel including polymorphic loci with high correlation with phenotype (low P value) is prepared. In single gene disorder testing, the presence or absence of mutations in single genes that directly cause diseases is analyzed. For the purpose of DTC genetic testing and single gene disorder testing, there is no restriction on the allele frequency of minor alleles, and genetic linkage (distance of single nucleotide polymorphism loci on chromosomes) is not considered. Therefore, the panel includes polymorphic loci related to minor alleles with extremely low allele frequency, and polymorphic loci that are close on chromosomes (have genetic linkage). As a result, even if PI (Paternity Index) is calculated by analyzing the panel, the CPI (Combined Paternity Index) calculated as the product of the PIs does not show a value effective as a judgment index for parentage testing, and therefore parentage testing cannot be performed (Non-Patent Document 1). Furthermore, since the panel constructed for the purpose of DTC genetic testing and single gene disorder testing is not designed for the purpose of evaluating chromosomal numerical abnormalities, chromosomal numerical abnormalities such as chromosome 13, chromosome 18, chromosome 21, chromosome X, and chromosome Y cannot be detected.

Although it is known to perform numerical abnormality analysis of chromosomes by SNV analysis, the SNV panel used here is not constructed for the purpose of parentage testing or DTC genetic testing, so parentage testing or DTC genetic testing or single gene disorder testing cannot be performed simultaneously with chromosomal numerical abnormality testing (Non-Patent Documents 3, 5).

As described above, since the panels used in conventional parentage testing, DTC genetic testing or single gene disorder testing, and chromosomal numerical abnormality testing are designed according to the purpose of each test, data related to SNVs acquired for the purpose of one test cannot be diverted for the purpose of other tests.

Furthermore, the concept of performing two or more tests based on data obtained by a single analysis had not been considered.

The present invention defines a set of single nucleotide polymorphism loci (set of targets of analysis) to be analyzed to address these issues. The present invention analyzes a set of targets of analysis that satisfies two or more conditions selected from the following (i) to (iii):

(i) The ratio of the number of single nucleotide polymorphism loci, which is known to have a phenotype to the total number of single nucleotide polymorphism loci included in the set of targets of analysis is 10% or more;

(ii) The ratio of the number of single nucleotide polymorphism loci, among the total number of single nucleotide polymorphism loci included in the set of targets of analysis, where the minor allele frequency is 1% to 50% and which are separated from each other by a distance of 20 kb or more on a chromosome, is 10% or more;

(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

The present invention analyzes single nucleotide polymorphism loci in the set of targets of analysis. This includes not only acquiring and analyzing data limited to single nucleotide polymorphism loci in a preset panel, but also performing genome-wide analysis and analyzing specific single nucleotide polymorphism loci from the obtained data.

Based on the data obtained from the analysis of the set of targets of analysis that satisfies the condition "(i) the ratio of the number of single nucleotide polymorphism loci, which is known to be associated with a phenotype to the total number of single nucleotide polymorphism loci included in the set of targets of analysis is 10% or more," it becomes possible to perform the Phenotype Analysis Step described later. When performing the Phenotype Analysis Step described later, the set of targets of analysis needs to satisfy condition (i).

Here, the "single nucleotide polymorphism locus known to be associated with a phenotype" refers to a single nucleotide polymorphism locus whose relationship with a phenotype is known based on statistical, medical, molecular biological, or other scientific grounds.

The single nucleotide polymorphism loci found so far are linked to related phenotypes, allele frequencies, mapping information, etc., and are stored in various databases. By searching these databases, single nucleotide polymorphism loci, which is known to be associated with phenotypes and their allele frequencies can be easily identified. Examples of such databases include the following:

dbSNP:

[https://www.ncbi.nlm.nih.gov/snp/](https://www.ncbi.nlm.nih.gov /snp/) SNPedia: [https://www.snpedia.com/](https://www.snpedia.com/)

A P value is known as a statistic that quantitatively indicates the relationship with a phenotype. The P value is a statistic that indicates the relationship between a mutation identified by GWAS and a phenotype, and the lower the value, the stronger the relationship. The P value can be calculated by applying a chi-square test to the odds ratio of individuals with and without the specific allele. In various databases, many single nucleotide polymorphism loci are registered in association with P values.

A single nucleotide polymorphism locus with a P value of 0.001 or less can be selected as one that satisfies condition (i). The P value of the single nucleotide polymorphism locus to be added to the set of targets of analysis that satisfies condition (i) is preferably $1 \times 10^{-4}$ or less, more preferably $1 \times 10^{-5}$ or less, still more preferably $1 \times 10^{-6}$ or less, even more preferably $1 \times 10^{-7}$ or less, and most preferably $1 \times 10^{-8}$ or less.

It is also possible to analyze multiple single nucleotide polymorphism loci that are relatively weakly associated with a phenotype (high P value) and that are involved in the same phenotype, and to assess them collectively as a polygenic risk score in the Phenotype Analysis Step described later.

Even if a P value is not shown, single nucleotide polymorphism loci, which is known to be associated with a phenotype based on reports such as academic papers can also be selected as single nucleotide polymorphism loci that satisfy condition (i) above.

To perform the Phenotype Analysis Step, the ratio of the number of single nucleotide polymorphism loci, which is known to be associated with a phenotype to the total number of single nucleotide polymorphism loci included in the set of targets of analysis is preferably 10% or more, more preferably 14% or more, still more preferably 20% or more, even more preferably 30% or more, yet more preferably 40% or more, still further preferably 50% or more, even further preferably 60% or more, and most preferably 70% or more, 80% or more, 90% or more, 95% or more, or 100%.

Based on the data obtained by analyzing a set of targets of analysis that satisfies the condition "(ii) the ratio of the number of single nucleotide polymorphism loci, among the total number of single nucleotide polymorphism loci included in the set of targets of analysis, where the minor allele frequency is 1% to 50% and which are separated from each other by a distance of 20 kb or more on a chromosome, is 10% or more," it becomes possible to perform the Parentage Testing Step described later. When performing the Parentage Testing Step described later, the set of targets of analysis needs to satisfy condition (ii).

In condition (ii), the minor allele frequency of the single nucleotide polymorphism locus is 1% to 50%. If necessary, the minor allele frequency of the single nucleotide polymorphism locus included in the set of targets of analysis that satisfies condition (ii) may be set to preferably 5% or more, more preferably 10% or more, still more preferably 20% or more, and even more preferably 30% or more. The higher the minor allele frequency is set, the fewer single nucleotide polymorphisms are needed to obtain CPI or CMI for effective parentage testing.

Alleles at multiple single nucleotide polymorphism loci whose loci are close to each other on the same chromosome are highly likely to be inherited by gametes as a single set (haplotype) during homologous recombination in meiosis. From the viewpoint of improving the accuracy of the Parentage Testing Step based on PI calculation, it is preferable that the single nucleotide polymorphism loci included in the set of targets of analysis do not show genetic linkage with each other.

Therefore, condition (ii) requires, in addition to the minor allele frequency being 1% to 50%, that a certain number of single nucleotide polymorphism loci are separated from each other by a distance of 20 kb or more on a chromosome.

The ratio of the number of single nucleotide polymorphism loci, among the total number of single nucleotide polymorphism loci included in the set of targets of analysis, where the minor allele frequency is 1% to 50% and which are separated from each other by a distance of 20 kb or more on a chromosome, is preferably 10% or more, more preferably 14% or more, still more preferably 20% or more, even more preferably 30% or more, yet more preferably 40% or more, still further preferably 50% or more, even further preferably 60% or more, and most preferably 70% or more, 80% or more, 90% or more, 95% or more, or 100%.

Based on the data obtained by analyzing a set of targets of analysis that satisfies the condition "(iii) the ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more," it becomes possible to perform the Calculation Step for Evaluating Chromosomal Numerical Abnormalities described later. When performing the Calculation Step for Evaluating Chromosomal Numerical Abnormalities described later, the set of targets of analysis needs to satisfy condition (iii).

The ratio of the number of single nucleotide polymorphism loci present on chromosome 13, chromosome 18, chromosome 21, chromosome X, and chromosome Y to the total number of single nucleotide polymorphism loci included in the set of targets of analysis is preferably 10% or more, more preferably 14% or more, and further preferably 14.7% or more.

Numerical abnormalities in chromosomes other than those listed above cause severe developmental abnormalities and lead to embryonic lethality. By designing the set of targets of analysis such that the ratio of single nucleotide polymorphism loci present on chromosome 13, chromosome 18, chromosome 21, chromosome X, and chromosome Y is maintained at a sufficient level, it is possible to effectively test for autosomal trisomies such as trisomy 21 (Down syndrome), trisomy 18 (Edwards syndrome), and trisomy 13 (Patau syndrome), and numerical abnormalities of sex chromosomes such as XXY (Klinefelter syndrome) and XYY (Jacob syndrome).

The possible embodiments corresponding to each condition satisfied by the set of targets of analysis are summarized below.

When using a set of targets of analysis that satisfies all the conditions (i) to (iii):

Embodiment I, in which the Phenotype Analysis Step, the Parentage Testing Step, and the Calculation Step for Evaluating Chromosomal Numerical Abnormalities are performed.

When analyzing a set of targets of analysis that satisfies the conditions (i) and (ii):

Embodiment II, in which the Phenotype Analysis Step and the Parentage Testing Step are performed.

When analyzing a set of targets of analysis that satisfies the conditions (i) and (iii):

Embodiment III, in which the Phenotype Analysis Step and the Calculation Step for Evaluating Chromosomal Numerical Abnormalities are performed.

When analyzing a set of targets of analysis that satisfies the conditions (ii) and (iii):

Embodiment IV, in which the Parentage Testing Step and the Calculation Step for Evaluating Chromosomal Numerical Abnormalities are performed.

Embodiment I includes Examples 1 and 5, which are described later.

Embodiment II includes Examples 2 and 6, which are described later.

Embodiment III includes Examples 3 and 7, which are described later.

Embodiment IV includes Examples 4 and 8, which are described later.

When adopting Embodiment I, the set of targets of analysis satisfies all of the conditions (i) to (iii) above. In a more preferred embodiment, the set of targets of analysis includes single nucleotide polymorphism loci that are known to be associated with a phenotype, have a minor allele frequency of 1% to 50%, are separated from each other by a distance of 20 kb or more on a chromosome, and present on chromosome 13, chromosome 18, chromosome 21, chromosome X, and chromosome Y.

The ratio of single nucleotide polymorphism loci satisfying these conditions to the total number of single nucleotide polymorphism loci constituting the set of targets of analysis is preferably 10% or more, more preferably 20% or more, still more preferably 30% or more, even more preferably 40% or more, yet more preferably 50% or more, still further preferably 60% or more, and most preferably 70% or more, 80% or more, 90% or more, 95% or more, or 100%.

When adopting Embodiment II, the set of targets of analysis satisfies the conditions (i) and (ii) above. In a more preferred embodiment, the set of targets of analysis includes single nucleotide polymorphism loci that are known to be associated with a phenotype, have a minor allele frequency of 1% to 50%, and are separated from each other by a distance of 20 kb or more on a chromosome.

The ratio of single nucleotide polymorphism loci satisfying these conditions to the total number of single nucleotide polymorphism loci constituting the set of targets of analysis is preferably 10% or more, more preferably 20% or more, still more preferably 30% or more, even more preferably 40% or more, yet more preferably 50% or more, still further preferably 60% or more, and most preferably 70% or more, 80% or more, 90% or more, 95% or more, or 100%.

When adopting Embodiment III, the set of targets of analysis satisfies the conditions (i) and (iii) above. In a more preferred embodiment, the set of targets of analysis includes single nucleotide polymorphism loci that are known to be associated with a phenotype and present on chromosome 13, chromosome 18, chromosome 21, chromosome X, and chromosome Y.

The ratio of single nucleotide polymorphism loci satisfying these conditions to the total number of single nucleotide polymorphism loci constituting the set of targets of analysis is preferably 10% or more, more preferably 20% or more, still more preferably 30% or more, even more preferably 40% or more, yet more preferably 50% or more, still further preferably 60% or more, and most preferably 70% or more, 80% or more, 90% or more, 95% or more, or 100%.

When adopting Embodiment IV, the set of targets of analysis satisfies the conditions (ii) and (iii) above. In a more preferred embodiment, the set of targets of analysis includes single nucleotide polymorphism loci that have a minor allele frequency of 1% to 50%, are separated from each other by a distance of 20 kb or more on a chromosome, and present on chromosome 13, chromosome 18, chromosome 21, chromosome X, and chromosome Y.

The ratio of single nucleotide polymorphism loci satisfying these conditions to the total number of single nucleotide polymorphism loci constituting the set of targets of analysis is preferably 10% or more, more preferably 20% or more, still more preferably 30% or more, even more preferably 40% or more, yet more preferably 50% or more, still further preferably 60% or more, and most preferably 70% or more, 80% or more, 90% or more, 95% or more, or 100%.

A list of single nucleotide polymorphism loci that are known to be associated with a phenotype, have a minor allele frequency of 1% to 50%, and are separated from each other by a distance of 20 kb or more on a chromosome is exemplified in Tables 9-1 to 9-14 at the end of the present specification. The ratio of the number of single nucleotide polymorphism loci present on chromosome 13, chromosome 18, chromosome 21, chromosome X, and chromosome Y to the total number of single nucleotide polymorphism loci exemplified in Tables 9-1 to 9-14 is 10% or more (71.2%). However, the present invention is not limited to the embodiment using a set of targets of analysis including the single nucleotide polymorphism loci exemplified in Tables 9-1 to 9-14.

The single nucleotide polymorphism loci with "-" in the "P-VALUE" item in Tables 9-1 to 9-14 are those whose relationship with phenotype is known but whose P values are not recorded in the database.

In one embodiment of the present invention, the ratio of single nucleotide polymorphism loci selected from the 361 single nucleotide polymorphism loci listed in Tables 9-1 to 9-14 is preferably 10% or more, more preferably 20% or more, still more preferably 30% or more, even more preferably 40% or more, yet more preferably 50% or more, still further preferably 60% or more, and most preferably 70% or more, 80% or more, 90% or more, 95% or more, or 100%. However, the present invention is not limited to such an embodiment.

The present invention includes a genotyping step based on data obtained by analyzing the set of targets of analysis described above. Specifically, a child's nucleic acid sample containing a nucleic acid with the genetic information of a child, and a father's nucleic acid sample containing a nucleic acid with the genetic information of a father and/or a mother's nucleic acid sample containing a nucleic acid with the genetic information of a mother are analyzed. By this analysis, data including allele sequence information at the multiple single nucleotide polymorphism loci included in the set of targets of analysis in the nucleic acids of each sample, and numerical values reflecting the region including the single nucleotide polymorphism loci and/or the relative or absolute abundance of the alleles are acquired, and genotyping is performed based on this data.

In the present specification, "genotyping" includes all methods for determining the genotype of a subject. "Genotyping" includes both direct analysis of genomic DNA and indirect determination of genotype by analyzing a cell-free nucleic acid sample.

In the genotyping step, a child's nucleic acid sample is analyzed. In addition to this, a paternal nucleic acid sample and/or a maternal nucleic acid sample (not containing a nucleic acid with genetic information of the child) may be analyzed.

As the "paternal nucleic acid sample," a sample usually used in genetic testing, such as a buccal mucosa cell sample collected from the father's buccal mucosa, can be adopted without limitation.

Similarly, as the "maternal nucleic acid sample" containing a nucleic acid with genetic information of the mother and not containing a nucleic acid with genetic information of the child, a sample usually used in genetic testing, such as a buccal mucosa cell sample collected from the mother's buccal mucosa, can be adopted without limitation.

The specific embodiment of the "child's nucleic acid sample" differs between the prenatal and postnatal cases.

As the child's nucleic acid sample containing genetic information of a prenatal child, a cell-free nucleic acid sample collected from maternal blood during pregnancy can be preferably exemplified. The cell-free nucleic acid sample is a sample in which a nucleic acid with genetic information of the mother and a nucleic acid with genetic information of the fetus in the mother's womb are mixed.

As the child's nucleic acid sample containing genetic information of a postnatal child, a sample usually used in genetic testing, such as a buccal mucosa cell sample collected from the child's buccal mucosa, can be adopted without limitation, as in the case of the "paternal nucleic acid sample" and the "maternal nucleic acid sample" described above.

As a means for analyzing the nucleic acid sample, any analysis method capable of detecting and distinguishing single nucleotide substitutions (SNVs) at polymorphic loci can be used without limitation. Specific examples include nucleotide sequence analysis, mass spectrometry, digital PCR, SNV microarray, and real-time PCR.

Next-generation sequencing (NGS) is a specific means of nucleotide sequence analysis. Next-generation sequencing is a sequencing method that allows for high-throughput parallel sequencing of clonally amplified molecules and single nucleic acid molecules. In the present invention, any NGS system may be adopted. Examples include pyrosequencing (such as GS Junior (Roche)), sequencing by synthesis using reversible dye terminators (such as MiSeq (Illumina)), sequencing by ligation (such as SeqStudio Genetic Analyzer (Thermo Fisher SCIENTIFIC)), ion semiconductor sequencing (such as Ion Proton System (Thermo Fisher SCIENTIFIC)), and sequencing by CMOS (complementary metal oxide semiconductor) chip (such as iSeq 100 System (Illumina)).

The number of sequenced reads aligned to a known reference sequence is called depth (also referred to as coverage). Data on coverage can be used for the evaluation of chromosomal numerical abnormalities, as will be described later.

In the case of amplicon analysis, data on the amount of amplicons and data on the total number of reads sequenced based on amplicons in a certain genomic region can also be used for the evaluation of chromosomal numerical abnormalities.

All of these data fall within the category of "numerical values reflecting the region including the single nucleotide polymorphism loci and/or the relative or absolute abundance of the alleles."

The sequence data read by the next-generation sequencer can be analyzed, and the number of reads of an allele having a specific sequence (specific SNVs) at a polymorphic locus can be interpreted as a signal indicating the presence of the allele.

When a unique molecular barcode (Unique Molecular Identifiers (UMI), Unique Molecular Tag (UMT)) that enables individual identification of nucleic acid molecules is linked to a nucleic acid fragment to be analyzed in the library preparation stage for next-generation sequencing, the number of UMTs detected for an allele with a specific sequence (specific SNVs) can be interpreted as a signal indicating the presence of the allele.

Analysis using a next-generation sequencer is particularly suitable in the present invention. When a next-generation sequencer is adopted as the analysis means in the present invention, in addition to the target sequencing method for specifically amplifying a polymorphic locus known in advance, DNA sequencing by non-target sequencing such as exon sequencing and GWAS can also be adopted.

In the case of target sequencing, a library can be prepared by a hybridization method or amplicon sequencing.

When non-target sequencing is adopted, a single nucleotide polymorphism locus having a predetermined profile of the present invention can be selected from the decoded nucleotide sequence, and qualitative or quantitative data on the single nucleotide polymorphism locus can be acquired.

Digital PCR is a method in which a sample is distributed into a large number of wells, each containing either one or no nucleic acid molecule, and PCR is performed individually. In wells containing the target sequence, PCR amplification proceeds and a fluorescent signal is detected, but in wells not containing the target sequence, PCR amplification does not proceed and no fluorescent signal is detected. After PCR, the presence (+) or absence (-) of signal amplification is determined in each well, and the number of wells with signal (+) is calculated as the copy number of the target.

If digital PCR is combined with a probe (such as a TaqMan® probe or a cycling probe) that can accurately discriminate mutations such as SNVs, fluorescence is observed only in wells where an allele having a specific sequence (specific SNVs) is amplified. By designing fluorescently labeled probes with different emission wavelengths for each allele, different alleles present at one polymorphic locus can be distinguished and detected by fluorescence color. The number of wells with a fluorescent signal (+) corresponding to a specific allele can be interpreted as a signal indicating the presence of the allele.

A microarray is an analytical technique that immobilizes nucleic acids with known sequences, such as DNA, DNA fragments, cDNA, oligonucleotides, RNA, or RNA fragments, as probes. These probes are arranged and immobilized in arrays containing hundreds to hundreds of thousands of sequences. When a nucleic acid with a complementary sequence hybridizes to a probe, it is detected using fluorescent labeling. A microarray for SNV typing is also called an SNV array. When multiple alleles are assumed at one locus, they can be distinguished and detected by separately immobilizing each allele. The fluorescence intensity at the point where a specific allele is immobilized can be interpreted as a signal indicating the presence of the allele.

Real-time PCR is a method in which fluorescence generated according to the amount of nucleic acid amplified by PCR is continuously monitored in real time by a spectrofluorophotometer. It is preferable to combine real-time PCR with a probe (such as a TaqMan® probe or a cycling probe) that can accurately discriminate mutations such as SNVs. By designing fluorescently labeled probes with different emission wavelengths for each allele, different alleles present at one polymorphic locus can be distinguished and detected by fluorescence color.

When attempting to obtain a data set using real-time PCR, it is preferable to adopt multiplex PCR from the viewpoint of improving measurement efficiency. Multiplex PCR is a method of amplifying multiple target sequences at once in one reaction system using multiple sets of primers.

In real-time PCR, the intensity of the fluorescence signal corresponding to a specific allele can be interpreted as a signal indicating the presence of the allele.

Mass spectrometry is an analytical method that determines the mass of ions and molecules by ionizing them and measuring their mass-to-charge ratio (m/z). Although it is originally a method for measuring the mass of molecules, if the mass of nucleic acid molecules prepared under specific conditions (such as when PCR is performed using specific primers or when nucleic acid molecules are cleaved with specific restriction enzymes) can be measured, the base sequence of the detected nucleic acid molecule can be identified by comparing the mass with a database. For this reason, mass spectrometry is widely applied to genotyping.

In mass spectrometry, the ion intensity at an m/z value specific to a base sequence including a specific allele can be interpreted as a signal indicating the presence of the allele.

In the genotyping step, data including allele sequence information at each single nucleotide polymorphism locus included in the set of targets of analysis, and quantitative values representing the region including the single nucleotide polymorphism locus and/or the relative or absolute abundance of the allele are acquired.

The "allele sequence information" here is used in the Parentage Testing Step and the Phenotype Analysis Step described later.

On the other hand, the "quantitative values representing the region including the single nucleotide polymorphism locus and/or the relative or absolute abundance of the allele" are numerical values reflecting the relative or absolute amount of nucleic acid or allele having a base sequence region including the single nucleotide polymorphism locus in the nucleic acid sample. These numerical values include the number of sequenced reads aligned to a known reference sequence, and in the case of amplicon analysis, data on the amount of amplicons and data on the total number of reads sequenced based on amplicons in a certain genomic region. These values are used in the Calculation Step for Evaluating Chromosomal Numerical Abnormalities described later.

The genotyping step of the present invention includes the following specific embodiments:

(a) Genotyping of the father based on the paternal nucleic acid sample;
(b) Genotyping of the mother based on the maternal nucleic acid sample;
(c) Genotyping of the child based on the child's nucleic acid sample collected from the child after birth;
(d) Genotyping of the fetus in the mother's womb based on the cell-free nucleic acid sample collected from the pregnant mother;
(e) Indirect genotyping of the mother based on the cell-free nucleic acid sample collected from the pregnant mother.

Genotyping of (a) to (c) may be suitably performed by a conventional method.

The "genotyping based on the child's nucleic acid sample" when the child is prenatal includes the genotyping of (d) to (f) based on the cell-free sample described above. Hereinafter, each of them will be described.

(d) Genotyping of the fetus during pregnancy based on the cell-free nucleic acid sample collected from the pregnant mother

The data obtained by analyzing the cell-free nucleic acid sample is a mixture of signals originating from the major component of nucleic acid derived from the mother and the minor component of nucleic acid derived from the fetus, so the genotype of the fetus cannot be directly identified. Therefore, when performing genotyping of the fetus based on the cell-free nucleic acid sample, first, the genotype of the mother is identified by genotyping the mother. Then, genotyping of the fetus can be performed by subtracting the information on the mother's genotype from the data obtained by analyzing the cell-free nucleic acid sample.

Of the two different alleles at a specific polymorphic locus, the allele with relatively higher signal intensity in the analysis of the cell-free nucleic acid sample is denoted as A, and the allele with relatively lower signal intensity is denoted as B. Genotyping of the fetus can be performed according to the following criteria using the ratio of the intensity of the signal indicating the presence of B (Freq. B) to the total signal intensity due to the alleles at the specific polymorphic locus as an index.

AA homozygote: Freq. B ≤ threshold value 1
AB heterozygote: threshold value 2 ≤ Freq. B ≤ threshold value 3

Here, the threshold values 1 to 3 can be appropriately set within a range satisfying the following equation:

0% ≤ threshold value 1 ≤ threshold value 2 ≤ threshold value 3 ≤ 45%

(e) Indirect genotyping of the mother based on the cell-free nucleic acid sample collected from the pregnant mother

Indirect genotyping of the mother may be conducted according to the following criteria using Freq. B as an evaluation metric.

AA homozygote: Freq. B ≤ threshold value 4

AB heterozygote: threshold value 5 ≤ Freq. B ≤ 50%

Here, the threshold values 4 and 5 can be appropriately set within a range satisfying the following equation:

$$0\% \le \text{threshold value } 4 \le \text{threshold value } 5 < 50\%$$

The method for indirect genotyping of the mother is not limited to the method described above. Any method for indirect genotyping of the mother that takes advantage of the fact that the abundance ratio of nucleic acid derived from the mother is generally higher than that of nucleic acid derived from the fetus in the cell-free nucleic acid sample falls within the scope of the present invention.

<2> Prenatal

Hereinafter, embodiments of the present invention when the child is prenatal will be described in detail.

When the child is prenatal, after the genotyping step, the genotyping of (a), (b), (d), and (e) described above can be performed. Unless otherwise specified, "genotyping of the mother" includes direct genotyping of the mother (b) and indirect genotyping of the mother (e).

When the child is prenatal, genotyping based on the analysis of the cell-free nucleic acid sample is performed, but a threshold value can be applied to the signal intensity and/or its ratio obtained by the analysis, and only signals exceeding the threshold value can be treated as valid.

Of the two different alleles at a specific polymorphic locus, the allele with relatively higher signal intensity in the analysis of the cell-free nucleic acid sample is denoted as A, and the allele with relatively lower signal intensity is denoted as B.

In this case, the signal may be treated as valid only when the signal intensity of B is equal to or higher than a predetermined threshold value. For example, when the analysis means is a next-generation sequencer, the threshold value can be set to preferably 5 amplicons or more, more preferably 15 amplicons or more, and further preferably 25 amplicons or more.

A threshold value may be applied to the ratio of the intensity of the signal indicating the presence of B to the total signal intensity due to the alleles at the specific polymorphic locus, and the signal may be treated as valid only when the ratio is equal to or higher than the threshold value. The threshold value for this ratio can be established at, for example, 0.1% or more, preferably 0.5% or more, and more preferably 1% or more.

When the child is prenatal, the steps that can be taken after the genotyping step are the Phenotype Analysis Step, the Parentage Testing Step, and the Calculation Step for Evaluating Chromosomal Numerical Abnormalities.

First, these steps will be described individually, and then more specific embodiments will be described.

<2-1> Phenotype Analysis Step (Prenatal)

The Phenotype Analysis Step in the prenatal stage is a step of analyzing the possibility of expression of a phenotype related to a minor allele at the single nucleotide polymorphism locus in one or more individuals selected from the fetus, father, and mother based on the result of the genotyping step. The Phenotype Analysis Step of the present invention corresponds to the analysis of the possibility of phenotype expression based on genotype performed in so-called DTC testing, as well as the evaluation of the likelihood of possessing a risk allele of a responsible gene for a single-gene disorder.

When performing the Phenotype Analysis Step, single nucleotide polymorphism loci included in the set of targets of analysis that satisfies condition (i) above are analyzed.

The relationship between genotype and phenotype compiled in GWAS studies is stored in databases such as the GWAS Catalog ([https://www.ebi.ac.uk/gwas/home] (https://www.ebi.ac.uk/gwas/ho me)). When a minor allele is detected at a single nucleotide polymorphism locus analyzed in the genotyping step, a phenotype related to the minor allele can be searched by inputting the ID of the single nucleotide polymorphism into the database.

The likelihood of phenotype expression related to a minor allele can be evaluated by taking into account the P value of the minor allele detected by analysis, the number of minor alleles related to the same phenotype, linkage disequilibrium, and the like.

If the father or mother possesses a risk allele, the phenotype associated with the risk allele is present, and the risk allele is inherited by the fetus, it can be estimated that the phenotype associated with the risk allele is more likely to be expressed in the fetus.

If the biological father of the fetus is uncertain, the advantageous effects described above can be obtained by performing the Phenotype Analysis Step after confirming the parent-child relationship between the putative father and the fetus through the Parentage Testing Step.

The targets of analysis in the Phenotype Analysis Step is one or more individuals selected from the child, father, and mother. In a preferred embodiment, two or more individuals among the child, father, and/or mother are analyzed.

<2-2> Parentage Testing Step (Prenatal)

The Parentage Testing Step in the prenatal stage is a step of determining the parent-child relationship between the putative father and the fetus in the mother's womb. When performing the Parentage Testing Step, single nucleotide polymorphism loci included in the set of targets of analysis that satisfies condition (ii) above are analyzed. Hereinafter, in the description of parentage testing, the father who is a party to the testing may be particularly referred to as a "putative father."

In parentage testing, a Paternity Index (PI) is calculated for each single nucleotide polymorphism locus based on the genotyping results of the putative father and the fetus.

More specifically, the PI is calculated for each single nucleotide polymorphism locus where the minor allele frequency is 1% to 50% and which is separated from other loci by a distance of 20 kb or more on a chromosome.

The method for calculating the Paternity Index is not specifically restricted, and any method may be adopted.

As an NIPPT method, any known method may be adopted, such as the method described in Patent Document 1, the method described in Non-Patent Document 2, and the method described in Non-Patent Document 3.

In addition to the known methods, for example, the method described below may be adopted.

First, a method of calculating PI based on the genotyping results of the putative father and the fetus will be described. In the present embodiment, genotyping of the mother is unnecessary, and parentage testing is performed based on the genotyping results of the putative father and the fetus.

In the case of the present embodiment, for example, the PI can be determined according to Table 1 or Table 2 below.

Table 1

| CASE | Genotype | | Paternity Index |
|------|----------|---|-----------------|
| | alleged father | fetus | PI |
| (I) | AA | AA | $\dfrac{1 - k_0}{(1 - k_0)a + k_0 b}$ |
| (II) | AA | AB | $\dfrac{k_b}{k_b a + (1 - k_b)b}$ |
| (III) | BB | AA | $\dfrac{k_0}{(1 - k_0)a + k_0 b}$ |
| (IV) | BB | AB | $\dfrac{1 - k_b}{k_b a + (1 - k_b)b}$ |
| (V) | AB | AA | $\dfrac{1}{2(1 - k_0)a + 2k_0 b}$ |
| (VI) | AB | AB | $\dfrac{1}{2k_b a + 2(1 - k_b)b}$ |

Table 2

| CASE | Genotype | | Paternity Index |
|---|---|---|---|
| | alleged father | fetus | PI |
| (I) | AA | AA | 1/a |
| (II) | AA | AB | kb |
| (III) | BB | AA | k0 |
| (IV) | BB | AB | 1/b |
| (V) | AB | AA | 0.5/a |
| (VI) | AB | AB | 0.5/b |

In Tables 1 and 2, the allele with relatively higher signal intensity in the analysis of the cell-free nucleic acid sample is denoted as A, and the allele with relatively lower signal intensity is denoted as B.

In Tables 1 and 2, $k_0$ is the false negative rate, kb is the false positive rate, a is the occurrence frequency of A, and b is the occurrence frequency of B.

The false negative rate is the probability that the true genotype is determined to be an AA homozygote even though the true genotype is an AB heterozygote but the signal of the valid allele B is not detected. The false negative rate is a parameter that fluctuates depending on the type of analysis method and measurement conditions, and can be specified in advance by testing. In the case of a specific next-generation sequencer, a false negative rate of about 0.002 to 0.13 has been reported on each platform (Non-Patent Document 4).

The false positive rate is the probability that the true genotype is determined to be an AB heterozygote even though the true genotype is an AA homozygote but the signal of allele B is erroneously detected. The false positive rate is a parameter that fluctuates depending on the type of analysis method and measurement conditions, and can be specified in advance by testing.

Next, the method for calculating PI based on the genotyping results of the mother, putative father, and fetus will be described. This embodiment allows for higher accuracy in testing because it takes into account the genotyping results of the mother.

In the case of the present embodiment, for example, the PI can be determined according to Tables 3 and 4 below.

Table 3

| CASE | Genotype | | | Paternity Index |
|---|---|---|---|---|
| | mother | alleged father | fetus | PI |
| (I) | AA | AA | AA | $\dfrac{1-k_0}{(1-k_0)a+k_0b}$ |
| (II) | AA | AA | AB | $\dfrac{k_b}{k_ba+(1-k_b)b}$ |
| (III) | AA | BB | AA | $\dfrac{k_0}{(1-k_0)a+k_0b}$ |
| (IV) | AA | BB | AB | $\dfrac{1-k_b}{k_ba+(1-k_b)b}$ |
| (V) | AA | AB | AA | $\dfrac{1}{2(1-k_0)a+2k_0b}$ |
| (VI) | AA | AB | AB | $\dfrac{1}{2k_ba+2(1-k_b)b}$ |

Table 4

| CASE | Genotype | | | Paternity Index |
|------|----------|-------|-------|-----------------|
| | mother | alleged father | fetus | PI |
| (I) | AA | AA | AA | 1/a |
| (II) | AA | AA | AB | kb |
| (III) | AA | BB | AA | k0 |
| (IV) | AA | BB | AB | 1/b |
| (V) | AA | AB | AA | 0.5/a |
| (VI) | AA | AB | AB | 0.5/b |

In Tables 3 and 4, the allele with relatively higher signal intensity in the analysis of the cell-free nucleic acid sample is denoted as A, and the allele with relatively lower signal intensity is denoted as B.

In Tables 3 and 4, $k_0$ is the false negative rate, kb is the false positive rate, a is the occurrence frequency of A, and b is the occurrence frequency of B.

The genotyping of the mother may be either direct genotyping of the mother (b) or indirect genotyping of the mother (e). For higher accuracy, it is preferable to adopt (b).

In Tables 1 to 4, the PI is calculated based on the two alleles A and B, but the single nucleotide polymorphism locus is not limited to a biallelic locus, and may be a triallelic or tetra-allelic locus. In order to simplify the determination, only biallelic loci can be chosen for PI calculation.

After determining the PI for each single nucleotide polymorphism locus by any of the methods described above, a Combined Paternity Index (CPI) may be calculated. The CPI is obtained as the product of PIs.

An embodiment can be adopted in which the parent-child relationship between the fetus and the putative father is confirmed when the CPI exceeds a reference value.

The reference value of CPI for confirming the parent-child relationship is preferably 100 or more, more preferably 1,000 or more, more preferably 5,000 or more, and further preferably 10,000 or more.

Even if a set of targets of analysis consisting of single nucleotide polymorphism loci with exceptionally low minor allele frequencies is analyzed, each PI will show a uniformly low value. Therefore, it is not possible to obtain a sufficient CPI that can be considered effective for parentage testing. In addition, even if the PI of polymorphic loci that have genetic linkage (distance between single nucleotide polymorphism loci on a chromosome) and are close to each other on a chromosome is calculated, it does not provide a reliable metric for parentage testing, so parentage testing cannot be performed. The single nucleotide polymorphism loci included in the set of targets of analysis that satisfies condition (ii) above are suitable for obtaining an effective CPI because the minor allele frequency is 1% to 50% and they are separated from each other by a distance of 20 kb or more on a chromosome.

After calculating the CPI, the Probability of Paternity (POP) may be calculated. The POP can be determined using the following equation. In the equation, P represents the prior probability of paternity.

$$\mathrm{POP} = \frac{CPI \times P}{CPI \times P + (1 - P)}$$

<2-3> Calculation Step for Evaluating Chromosomal Numerical Abnormalities (Prenatal)

The Calculation Step for Evaluating Chromosomal Numerical Abnormalities in the prenatal stage is a step of evaluating the presence or absence of numerical abnormalities in chromosome 13, chromosome 18, chromosome 21, chromosome X, and chromosome Y in a subject based on numerical values representing the region including the single nucleotide polymorphism locus and/or the relative or absolute abundance of the allele included in the data obtained by analyzing the cell-free nucleic acid sample.

In this step, the presence or absence of numerical abnormalities in chromosomes can be evaluated. Specifically, trisomies such as trisomy 21 (Down syndrome), trisomy 18 (Edwards syndrome), and trisomy 13 (Patau syndrome), Klinefelter syndrome (47, XXY), Jacob syndrome (XYY syndrome), and very rarely, the presence or absence of tetrasomy can be evaluated.

When performing the Calculation Step for Evaluating Chromosomal Numerical Abnormalities, the set of targets of analysis that satisfies condition (iii) above is analyzed.

The subject of evaluation in this step is one or more individuals selected from the fetus, father, and mother.

When the fetus is the subject of evaluation, the cell-free nucleic acid sample serves as the specimen under examination.

When the father is the subject of evaluation, the paternal nucleic acid sample serves as the specimen under examination, and when the mother is the subject of evaluation, the maternal nucleic acid sample serves as the specimen under examination.

The Calculation Step for Evaluating Chromosomal Numerical Abnormalities can be performed by a conventional method using numerical values representing the region including the single nucleotide polymorphism locus and/or the relative or absolute abundance of the allele.

When performing analysis using a next-generation sequencer, data such as the number of reads (sequence depth), data on the amount of amplicons, and the total number of reads sequenced based on amplicons in a certain genomic region can be used as the numerical values.

Hereinafter, two methods for calculating an index for quantitatively evaluating chromosomal numerical abnormalities will be exemplified. For the sake of simplification, "numerical values representing the region including the single nucleotide polymorphism locus and/or the relative or absolute abundance of the allele" will be referred to as "the relevant numerical value."

First, a method for calculating the Z-score based on the ratio of the relevant numerical value of the target chromosome to the entire chromosome will be described (for details, see Non-Patent Document 5). In this method, the Z-score (Z chrN) for chromosome N is calculated based on the following equation (1).

$$Z_{chrN}$$
$$= \frac{proportion\ of\ chr\ N - mean\ proportion\ of\ chr\ N\ in\ reference\ disomic\ population}{SD\ of\ proportion\ of\ chr\ N\ in\ reference\ disomic\ population}$$

...equation (1)

In equation (1), "proportion of chr N" is the ratio of the relevant numerical value of chromosome N to the relevant numerical value of all chromosomes (excluding chromosomes 13, 18, and 21) when the nucleic acid sample to be tested is analyzed.

"Mean of proportion of chr N in reference disomic population" is the average of the ratio of the relevant numerical value of chromosome N to all chromosomes when a plurality of standard samples including a normal number of chromosomes (disomy) are analyzed.

"SD of proportion of chr N in reference disomic population" is the standard deviation of the ratio of the relevant numerical value of chromosome N to all chromosomes when a plurality of standard samples including a normal number of chromosomes (disomy) are analyzed.

It may be concluded that there is a chromosomal numerical abnormalities when the Z-score for the target chromosome in the nucleic acid sample to be tested deviates from the Z-score for the target chromosome when a standard sample including a normal number of chromosomes (disomy) is analyzed.

Next, a method for calculating the normalized chromosome value (NCV) for each chromosome, such as chromosome 21, chromosome 18, chromosome 13, chromosome X, and chromosome Y, with respect to a reference chromosome will be described (for details, see Non-Patent Document 6).

The NCV (NCV chrN by chrM) for chromosome N with respect to reference chromosome M can be calculated using the following equation (2).

$$NCV_{chrN\ by\ chr\ M}$$
$$= \frac{Normalized\ proportion\ of\ chr\ N\ by\ chr\ M - mean\ normalized\ proportion\ of\ chr\ N\ in\ reference\ disomic\ population}{SD\ of\ normalized\ proportion\ of\ chr\ N\ in\ reference\ disomic\ population}$$

...equation (2)

In equation (2), "Normalized proportion of chr N by chr M" is the ratio of the relevant numerical value of chromosome N to the relevant numerical value of chromosome M when the nucleic acid sample to be tested is analyzed.

"Mean normalized proportion of chr N in reference disomic population" is the average of the ratio of the relevant numerical value of chromosome N to chromosome M when a plurality of standard samples including a normal number of chromosomes (disomy) are analyzed.

"SD of proportion of chr N in reference disomic population" is the standard deviation of the ratio of the relevant numerical value of chromosome N to chromosome M when a plurality of standard samples including a normal number of chromosomes (disomy) are analyzed.

In an actual test, which chromosome is selected as chromosome M in order to calculate the NCV of the target chromosome N is determined by performing a preliminary test.

That is, a standard sample including a normal number of chromosomes (disomy) and a standard sample having a numerical abnormality in chromosome N are analyzed, and NCV chrN by chrM is calculated when chromosomes 1 to 22 are selected as chromosome M.

At this time, the formula of NCV chrN by chrM related to the combination of N and M with the largest difference in NCV score between the standard sample of disomy and the standard sample having a numerical abnormality is adopted for the test.

The following are embodiments of the present invention that can be adopted when the child is in the prenatal stage.

Embodiment in which a set of targets of analysis satisfying conditions (i) to (iii) is analyzed:

- Embodiment in which the Phenotype Analysis Step, the Parentage Testing Step, and the Calculation Step for Evaluating Chromosomal Numerical Abnormalities are performed (Example 1).

Embodiment in which a set of targets of analysis satisfying conditions (i) and (ii) is analyzed:

- Embodiment in which the Phenotype Analysis Step and the Parentage Testing Step are performed (Example 2).

Embodiment in which a set of targets of analysis satisfying conditions (i) and (iii) is analyzed:

- Embodiment in which the Phenotype Analysis Step and the Calculation Step for Evaluating Chromosomal Numerical Abnormalities are performed (Example 3).

Embodiment in which a set of targets of analysis satisfying conditions (ii) and (iii) is analyzed:

- Embodiment in which the Parentage Testing Step and the Calculation Step for Evaluating Chromosomal Numerical Abnormalities are performed (Example 4).

Hereinafter, Examples 1 to 4 that can be adopted when the child is in the prenatal stage will be described in detail.

<2-4> Example 1 (Combination of Phenotype Analysis Step, Parentage Testing Step, and Calculation Step for Evaluating Chromosomal Numerical Abnormalities in the Prenatal Stage)

Example 1 is an embodiment including three testing processes: a Parentage Testing Step, a Calculation Step for Evaluating Chromosomal Numerical Abnormalities, and a Phenotype Analysis Step, in addition to the Genotyping Step.

In Example 1, a set of targets of analysis including single nucleotide polymorphism loci that satisfy all of the following conditions (i) to (iii) is analyzed.

(i) The ratio of the number of single nucleotide polymorphism loci, which is known to have a phenotype to the total number of single nucleotide polymorphism loci included in the set of targets of analysis is 10% or more.
(ii) The ratio of the number of single nucleotide polymorphism loci that have a minor allele frequency of 1% to 50% and are separated from each other by a distance of 20 kb or more on a chromosome to the total number of single nucleotide polymorphism loci included in the set of targets of analysis is 10% or more.
(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

By analyzing the set of targets of analysis including single nucleotide polymorphism loci that satisfy all of the conditions (i) to (iii), data that can be used in any of the three testing processes of the Phenotype Analysis Step, the Parentage Testing Step, and the Calculation Step for Evaluating Chromosomal Numerical Abnormalities can be acquired.

Next, a genotyping step of analyzing at least a father's nucleic acid sample containing genetic information of the putative father and a cell-free nucleic acid sample is performed for each single nucleotide polymorphism locus included in the set of targets of analysis.

In the genotyping step, genotyping of the putative father (genotyping of (a) above) and genotyping of the fetus (genotyping of (d) above) are performed. In Example 1, genotyping of the mother (genotyping of (b) and/or (e) above) may further be performed.

In the genotyping step, an embodiment may be adopted in which only genotyping of the putative father (a) and genotyping of the fetus (d) are performed based on the analysis of only the father's nucleic acid sample and the cell-free nucleic acid sample, and genotyping of the mother is not performed.

Such an embodiment is advantageous as it allows for easy execution of the Parentage Testing Step.

In the genotyping step, an embodiment can be implemented in which genotyping of the putative father (a), genotyping of the fetus (d), and genotyping of the mother (b) are performed based on the analysis of the father's nucleic acid sample, the mother's nucleic acid sample, and the cell-free nucleic acid sample.

Such an embodiment is expected to enhance the accuracy of the Parentage Testing Step by performing genotyping of the mother.

In Example 1, based on the results of the genotyping step, a Parentage Testing Step for determining whether a parent-child relationship exists between the putative father and the fetus and a Phenotype Analysis Step are performed.

In addition, a Calculation Step for Evaluating Chromosomal Numerical Abnormalities is performed based on the data obtained by the analysis of the nucleic acid samples.

Unless otherwise specified, "parent-child relationship" in this specification refers to a biological parent-child relationship.

<2-5> Example 2 (Combination of Phenotype Analysis Step and Parentage Testing Step in the Prenatal Stage)

Example 2 is an embodiment including a Phenotype Analysis Step and a Parentage Testing Step. By performing parentage testing, an improvement in the accuracy of the Phenotype Analysis Step can be obtained.

In Example 2, a set of targets of analysis containing single nucleotide polymorphism loci that satisfy the following conditions (i) and (ii) is analyzed.

(i) The ratio of the number of single nucleotide polymorphism loci, which is known to be associated with a phenotype to the total number of single nucleotide polymorphism loci included in the set of targets of analysis is 10% or more.
(ii) The ratio of the number of single nucleotide polymorphism loci with a minor allele frequency of 1% to 50% and separated from each other by a distance of 20 kb or more on a chromosome to the total number of single nucleotide polymorphism loci included in the set of targets of analysis is 10% or more.

The explanation of Example 1 can be directly applied to the embodiment of genotyping performed on the set of targets of analysis that satisfies the above conditions.

By analyzing the set of targets of analysis containing single nucleotide polymorphism loci that satisfy the conditions (i) and (ii), the set of targets of analysis in Example 2 can acquire data that can be used in both of the two testing processes, the Phenotype Analysis Step and the Parentage Testing Step.

In Example 2, based on the results of the genotyping step, a Parentage Testing Step for determining whether a parent-child relationship exists between the putative father and the fetus and a Phenotype Analysis Step are performed.

In Examples 1 and 2, the Phenotype Analysis Step may be performed when the parent-child relationship between the putative father and the fetus is confirmed in the Parentage Testing Step, and the Phenotype Analysis Step may not be performed when the parent-child relationship is not confirmed.

Of course, the Phenotype Analysis Step can be performed regardless of the result of the Parentage Testing Step.

In this embodiment, non-invasive prenatal parentage testing and phenotype analysis can be performed based on data obtained by a single sample analysis.

In this embodiment, by confirming the parent-child relationship between the fetus and the father in the Parentage Testing Step, it becomes possible to determine whether the minor allele inherited by the fetus is derived from the father or the mother in the Phenotype Analysis Step.

Thus, when the father or mother possesses a risk allele and the risk allele is also inherited by the fetus, it can be inferred that there is a higher possibility that the phenotype related to the risk allele will be expressed in the fetus.

In addition, when the father or mother possesses a risk allele, and the phenotype related to the risk allele is expressed, and the risk allele is also inherited by the fetus, it can be inferred that there is a higher possibility that the phenotype related to the risk allele will be expressed in the fetus.

By confirming the parent-child relationship between the putative father and the fetus in the Parentage Testing Step and performing the Phenotype Analysis Step, the aforementioned advantageous effects can be obtained.

Since the genotype of the prenatal fetus is determined based on cell-free nucleic acids containing a small amount of fetal-derived nucleic acids, even if it is determined in the genotyping step that the fetus has a risk allele, the determination can be said to include a degree of uncertainty.

Since Examples 1 and 2 include a Parentage Testing Step, this problem can be solved and the accuracy of the Phenotype Analysis Step can be improved.

Specifically, in Examples 1 and 2, when the parent-child relationship can be confirmed by the Parentage Testing Step, the possibility that the fetus possesses a risk allele can be determined more accurately based on the following criteria, and the accuracy of the Phenotype Analysis Step can be improved.

(Criteria)

1) When one parent is homozygous for the risk allele: The fetus is determined to inherit the risk allele with 100% certainty.

2) When one parent is heterozygous for the risk allele: The fetus is determined to have a higher probability of inheriting the risk allele.

3) When both parents are heterozygous for the risk allele: The fetus is determined to have a higher probability of inheriting the risk allele than in case (2).

A more specific application case will be described.

Let P1 (%) be the estimated probability of the fetus inheriting a risk allele when a signal suggesting the presence of a fetal-derived risk allele is obtained by analyzing a cell-free nucleic acid sample.

Then, when the following criteria are met, P1 (%) is adjusted, and it is evaluated that the probability of the fetus inheriting the risk allele is P2 (%).

(Criteria)

1) When one parent is homozygous for the risk allele:

$$P2 = 100\%$$

2) When one parent is heterozygous for the risk allele:

$$P2 = P1 + (P1 \times 0.5)$$

(P2 is 1.5 times P1)

3) When both parents are heterozygous for the risk allele:

$$P2 = P1 + (P1 \times 0.75)$$

(P2 is 1.75 times P1)

However, if the right side of the equation in 2) and 3) exceeds 100%, P2 is set to 100%.

The method for calculating P1 is not particularly limited.

For example, the reliability value of a signal suggesting the presence of a risk allele in the fetus may be calculated by the method according to Patent No. 7121440, and this may be regarded as P1.

In addition, when a signal suggesting the presence of a risk allele in the fetus is detected, a predetermined constant probability may be predefined as P1.

<2-6> Example 3 (Combination of Calculation Step for Evaluating Chromosomal Numerical Abnormalities and Phenotype Analysis Step in the Prenatal Stage)

Example 3 is an embodiment including both a Calculation Step for Evaluating Chromosomal Numerical Abnormalities and a Phenotype Analysis Step in addition to the genotyping step.

According to Example 3, based on data obtained by a single sample analysis, it is possible to perform two types of testing processes, a Calculation Step for Evaluating Chromosomal Numerical Abnormalities and a Phenotype Analysis Step, at once, which provides an advantageous effect.

In Example 3, a set of targets of analysis including single nucleotide polymorphism loci that satisfy the following conditions (i) and (iii) is analyzed.

(i) The ratio of the number of single nucleotide polymorphism loci, which is known to have a phenotype to the total number of single nucleotide polymorphism loci included in the set of targets of analysis is 10% or more.

(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

By analyzing a set of targets of analysis including single nucleotide polymorphism loci that satisfy the conditions (i) and (iii), it is possible to acquire data that can be used for both the Phenotype Analysis Step and the Calculation Step for Evaluating Chromosomal Numerical Abnormalities.

This allows Example 3 to perform both the Calculation Step for Evaluating Chromosomal Numerical Abnormalities and the Phenotype Analysis Step.

In Example 3, genotyping of the test subject is performed using the genomic DNA or cell-free nucleic acid (cfDNA) of the test subject, and phenotype analysis is conducted.

By performing both of the two steps, the Calculation Step for Evaluating Chromosomal Numerical Abnormalities and the Phenotype Analysis Step, it is possible to more accurately determine the possibility of expression of a phenotype related to a minor allele in consideration of chromosomal numerical abnormalities.

Assume a normal allele A and a risk allele B at a certain locus.

If the risk allele B corresponds to a loss-of-function mutation or a dominant-negative mutation, the severity of the disease caused by the risk allele B may increase when the chromosome has a numerical abnormality (trisomy) and carries the alleles in the combination of (A, B, B), compared to when the chromosome normally has a pair of chromosomes and is heterozygous for (A, B).

By performing both of the two steps, the Calculation Step for Evaluating Chromosomal Numerical Abnormalities and the Phenotype Analysis Step, it becomes possible to assess the likelihoods as described above.

The specific embodiments of the Calculation Step for Evaluating Chromosomal Numerical Abnormalities and the Phenotype Analysis Step in Example 3 can be applied as described above.

<2-6> Example 3 (Combination of Calculation Step for Evaluating Chromosomal Numerical Abnormalities and Phenotype Analysis Step in the Prenatal Stage)

Example 3 is an embodiment including both a Calculation Step for Evaluating Chromosomal Numerical Abnormalities and a Phenotype Analysis Step in addition to the genotyping step.

According to Example 3, based on data obtained by a single sample analysis, it is possible to perform two types of testing processes, a Calculation Step for Evaluating Chromosomal Numerical Abnormalities and a Phenotype Analysis Step, at once, which provides an advantageous effect.

In Example 3, a set of targets of analysis including single nucleotide polymorphism loci that satisfy the following conditions (i) and (iii) is analyzed.

(i) The ratio of the number of single nucleotide polymorphism loci, which is known to have a phenotype to the total number of single nucleotide polymorphism loci included in the set of targets of analysis is 10% or more.
(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

By analyzing a set of targets of analysis including single nucleotide polymorphism loci that satisfy the conditions (i) and (iii), it is possible to acquire data that can be used for both the Phenotype Analysis Step and the Calculation Step for Evaluating Chromosomal Numerical Abnormalities.

This allows Example 3 to perform both the Calculation Step for Evaluating Chromosomal Numerical Abnormalities and the Phenotype Analysis Step.

In Example 3, genotyping of the test subject is performed using the genomic DNA or cell-free nucleic acid (cfDNA) of the test subject, and phenotype analysis is conducted.

By performing both of the two steps, the Calculation Step for Evaluating Chromosomal Numerical Abnormalities

and the Phenotype Analysis Step, it is possible to more accurately determine the possibility of expression of a phenotype related to a minor allele in consideration of chromosomal numerical abnormalities.

Assume a normal allele A and a risk allele B at a certain locus.

If the risk allele B corresponds to a loss-of-function mutation or a dominant-negative mutation, the severity of the disease caused by the risk allele B may increase when the chromosome has a numerical abnormality (trisomy) and carries the alleles in the combination of (A, B, B), compared to when the chromosome normally has a pair of chromosomes and is heterozygous for (A, B).

By performing both of the two steps, the Calculation Step for Evaluating Chromosomal Numerical Abnormalities and the Phenotype Analysis Step, it becomes possible to assess the likelihoods as described above.

The specific embodiments of the Calculation Step for Evaluating Chromosomal Numerical Abnormalities and the Phenotype Analysis Step in Example 3 can be applied as described above.

<2-7> Example 4 (Combination of Parentage Testing Step and calculation Step for Evaluating Chromosomal Numerical Abnormalities in the Prenatal Stage)

Example 4 is an embodiment including a Parentage Testing Step and a Calculation Step for Evaluating Chromosomal Numerical Abnormalities in addition to the genotyping step.

According to Example 4, parentage testing and evaluation of chromosomal numerical abnormalities can be performed based on data obtained by a single sample analysis.

In Example 4, a set of targets of analysis containing single nucleotide polymorphism loci that satisfy the following conditions (ii) and (iii) is analyzed.

(ii) The ratio of the number of single nucleotide polymorphism loci with a minor allele frequency of 1% to 50% and separated from each other by a distance of 20 kb or more on a chromosome to the total number of single nucleotide polymorphism loci included in the set of targets of analysis is 10% or more.

(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

By analyzing the set of targets of analysis, data that can be used for both the Parentage Testing Step and the Calculation Step for Evaluating Chromosomal Numerical Abnormalities can be obtained.

This allows Example 4 to perform both the Parentage Testing Step and the Calculation Step for Evaluating Chromosomal Numerical Abnormalities.

When the parent-child relationship is established as a result of performing the Parentage Testing Step, the accuracy of the evaluation by the Calculation Step for Evaluating Chromosomal Numerical Abnormalities can be enhanced.

For example, assume a case where both the father and the mother have a different type of allele at a certain single nucleotide polymorphism locus (mother AA, father BB).

When the biological parent-child relationship between the fetus and the father is established, it is determined that the fetal genotype at the single nucleotide polymorphism locus is AB heterozygous.

That is, fetal nucleic acids can be identified by a signal suggesting the presence of allele B obtained by analyzing a cell-free nucleic acid sample, and the content of fetal nucleic acids can be accurately evaluated.

This enhances the accuracy of chromosomal numerical abnormality evaluation.

This effect can be obtained not only when Example 4 is adopted, but also when Example 1 is adopted.

The description of Example 1 can be directly applied to the embodiment of the genotyping step in Example 4.

In addition, the above description can be directly applied to the specific embodiments of the Parentage Testing Step and the Calculation Step for Evaluating Chromosomal Numerical Abnormalities in Example 4.

<3> Postnatal

Hereinafter, embodiments of the present invention in the postnatal stage will be described in detail.

When the child is postnatal, in the genotyping step, the genotyping of (a) to (c) described above can be performed.

Even after the child is born, the steps that can be taken after the genotyping step are the Parentage Testing Step, the calculation step for Evaluating Chromosomal Numerical Abnormalities, and the Phenotype Analysis Step.

First, these steps will be described individually, and then more specific embodiments will be described.

<3-1> Phenotype Analysis Step (Postnatal)

The Phenotype Analysis Step in the postnatal stage will be described. The Phenotype Analysis Step is a step of evaluating the possibility of expression of the phenotype related to the minor allele at the single nucleotide polymorphism locus in one or more selected from the postnatal child, the father, and the mother based on the result of the genotyping step.

When performing the Phenotype Analysis Step, the set of targets of analysis satisfying the condition (i) above is analyzed.

When a minor allele is detected at a single nucleotide polymorphism locus analyzed in the genotyping step, a phenotype related to the minor allele is searched by inputting the ID of the single nucleotide polymorphism into a database in which GWAS data is accumulated.

The possibility of expression of a phenotype related to a minor allele can be evaluated in consideration of the P-value of the minor allele detected by analysis, the number of minor alleles related to the same phenotype, linkage disequilibrium, and the like.

<3-2> Parentage Testing Step (Postnatal)

The Parentage Testing Step in the postnatal stage is a step of determining the presence or absence of a parent-child relationship between the putative father and the child and/or the presence or absence of a parent-child relationship between the putative mother and the child based on the result of the genotyping step.

When performing the Parentage Testing Step, the set of targets of analysis satisfying the condition (ii) above is analyzed.

More specifically, the PI or MI is determined for each single nucleotide polymorphism locus where the minor allele frequency is 1% to 50% and which are separated from each other by a distance of 20 kb or more on a chromosome.

The specific embodiment of the Parentage Testing Step is not particularly limited. Any known testing method can be adopted.

Hereinafter, an embodiment in which paternity testing is performed and an embodiment in which maternity testing is performed will be described.

First, an embodiment in which paternity testing is performed based on the result of genotyping of the putative father and the child will be described.

In the present embodiment, genotyping of the mother is not required, and paternity testing is performed based on the genotyping results of the putative father and the child.

In the case of the present embodiment, for example, the PI can be determined according to Table 5 below.

Table 5

| CASE | Genotype | | Paternity Index |
|---|---|---|---|
| | alleged father | child | PI |
| (I) | AA | AA | 1/a |
| (II) | AA | AB | 0.5/a |
| (III) | BB | AA | $\mu/\beta$ |
| (IV) | AB | AA | 0.5/a |
| (V) | AB | AB | (a+b) 4ab |

In Table 5, $\mu$ is the mutation rate, and a and b represent the occurrence frequencies of alleles A and B, respectively.

$\beta$ is the Mean Power of Exclusion value of allele B.

Next, among the approaches to conducting paternity testing, an approach to conducting parentage testing based on the genotyping results of the mother, the putative father, and the child will be described.

In the case of this embodiment, for example, the PI can be calculated according to Table 6 below.

Table 6

| CASE | Genotype | | | Paternity Index |
|---|---|---|---|---|
| | mother | alleged father | child | PI |
| (I) | AA | AA | AA | 1/a |

(continued)

| CASE | Genotype | | | Paternity Index |
|------|--------|----------------|-------|-----------------|
|      | mother | alleged father | child | PI              |
| (II)  | AA | AA | AB | μ/β |
| (III) | AA | BB | AA | μ/α |
| (IV)  | AA | BB | AB | 1/b |
| (V)   | AA | AB | AA | 0.5/a |
| (VI)  | AA | AB | AB | 1/ (a+b) |

In Table 6, μ is the mutation rate, and a and b represent the occurrence frequencies of alleles A and B, respectively.

α is the Mean Power of Exclusion of allele A, and β is the Mean Power of Exclusion of allele B.

Although the PI is calculated based on the two alleles A and B in Tables 5 and 6, the single nucleotide polymorphism locus is not limited to a biallelic locus and may be a triallelic or tetra-allelic locus.

In order to simplify the determination, only biallelic loci may be selected for PI calculation.

After obtaining the PI for each single nucleotide polymorphism locus by any of the methods described above, a combined paternity index (CPI) may be calculated.

The CPI is obtained as the total product of the PIs.

An embodiment can be adopted in which the parent-child relationship between the fetus and the putative father is confirmed when the CPI exceeds a reference value.

The reference value of CPI for confirming the parent-child relationship is preferably 100 or more, more preferably 1,000 or more, more preferably 5,000 or more, and further preferably 10,000 or more.

In the case of an embodiment in which the PI is calculated using the equation in Table 5 based on the genotyping of the putative father and the child, and the CPI is thereby calculated,

the number of predetermined single nucleotide polymorphism loci included in the targets of analysis set as a basis for analysis is preferably 20 or more, more preferably 30 or more, further preferably 40 or more, further preferably 50 or more, and further preferably 60 or more.

By setting the number of predetermined single nucleotide polymorphism loci included in the targets of analysis set within the above range, a CPI sufficient to confirm parentage testing can be obtained.

Additionally, for more reliable confirmation of parentage testing, the number of predetermined single nucleotide polymorphism loci included in the targets of analysis set as a basis for analysis may preferably be 200 or more, more preferably 250 or more, and further preferably 550 or more.

In the case of an embodiment in which the PI is calculated using the equation in Table 6 based on the genotyping of the mother, the putative father, and the child,

and the CPI is thereby calculated, the number of predetermined single nucleotide polymorphism loci included in the set of targets of analysis as a basis for analysis is preferably 20 or more, more preferably 30 or more, further preferably 40 or more, and further preferably 50 or more.

By setting the number of predetermined single nucleotide polymorphism loci included in the set of targets of analysis within the above range, a CPI sufficient to confirm parentage testing can be obtained.

Additionally, for more reliable confirmation of parentage testing, the number of predetermined single nucleotide polymorphism loci included in the set of targets of analysis as a basis for analysis may preferably be 60 or more, more preferably 100 or more, and further preferably 200 or more.

Even if a set of single nucleotide polymorphism loci with extremely low minor allele frequencies is analyzed, each PI will show a uniformly low value.

Therefore, it is not possible to obtain a sufficient CPI that can be said to be effective for parentage testing.

The single nucleotide polymorphism loci included in the set of targets of analysis used in the present invention have a minor allele frequency of 1% to 50%, which is suitable for obtaining an effective CPI.

After calculating the CPI, the Probability of Paternity (POP) may be determined.

The POP can be calculated using the following equation.

$$POP = \frac{CPI \times P}{CPI \times P + (1 - P)}$$

Next, an embodiment in which maternity testing is performed will be described.

First, an embodiment in which maternity testing is performed based on the genotyping results of the putative mother and the child will be described.

In the case of this embodiment, for example, the maternity index (MI) can be calculated as shown in Table 7 below, based on the calculation formula for PI presented in Table 5.

Table 7

| CASE | Genotype | | Paternity Index |
|------|----------------|-------|-----------------|
| | alleged mather | child | PI |
| (I) | AA | AA | 1/a |
| (II) | AA | AB | 0.5/a |
| (III) | BB | AA | $\mu/\beta$ |
| (IV) | AB | AA | 0.5/a |
| (V) | AB | AB | (a+b) 4ab |

Further, the combined maternity index (CMI) of the motherchild relationship can be calculated by multiplying the MIs.

Furthermore, the probability of maternity (POM) can be calculated according to the above POP calculation formula.

Next, an embodiment in which maternity testing is performed based on the genotyping results of the father, the putative mother, and the child will be described.

In this case, it is assumed that the father is the biological father of the child, and maternity testing is performed.

The MI calculation in this case can be adapted accordingly by replacing "putative father" in Table 6 above with "putative mother" and "mother" with "father".

Specifically, it can be calculated as shown in Table 8.

Table 8

| CASE | Genotype | | | Paternity Index |
|------|----------------|--------|-------|-----------------|
| | alleged mother | father | child | PI |
| (I) | AA | AA | AA | 1/a |
| (II) | AA | AA | AB | $\mu/\beta$ |
| (III) | AA | BB | AA | $\mu/\alpha$ |
| (IV) | AA | BB | AB | 1/b |
| (V) | AA | AB | AA | 0.5/a |
| (VI) | AA | AB | AB | 1/ (a+b) |

<3-3> Index Calculation Step for Chromosomal Copy Number Abnormality Evaluation (Postnatal)

The Index Calculation Step for Chromosomal Copy Number Abnormality Evaluation after birth is a step of calculating an index for evaluating the presence or absence of numerical abnormalities of chromosome 13, chromosome 18, chromosome 21, chromosome X, and chromosome Y in one or more selected from the postnatal child, father, and mother.

When performing the Index Calculation Step for Chromosomal Copy Number Abnormality Evaluation, the set of targets of analysis that satisfies the condition (iii) above is analyzed.

As for the specific embodiment, the description in "<2-2>" regarding prenatal can be applied as it is.

The difference is that the sample used for the analysis of chromosomal numerical abnormalities of the child is a child's nucleic acid sample collected from the child after birth, whereas it was a circulating cell-free nucleic acid sample before birth.

Since evaluation is performed using the postnatal child's nucleic acid sample, if the child has a chromosomal numerical abnormality, a very clear difference occurs in the calculated Z-score or NCV compared to the case where a circulating cell-free nucleic acid sample is used.

The following embodiments of the present invention can be adopted when the child is postnatal.

A form of analyzing a set of targets of analysis that satisfies all the conditions (i) to (iii):

- Embodiment in which a Phenotype Analysis Step, a Parentage Testing Step, and an Index Calculation Step for Chromosomal Copy Number Abnormality Evaluation are performed (Example 5).

A form of analyzing a set of targets of analysis that satisfies the conditions (i) and (ii):

- Embodiment in which a Phenotype Analysis Step and a Parentage Testing Step are performed (Example 6).

A form of analyzing a set of targets of analysis that satisfies the conditions (i) and (iii):

- Embodiment in which a Phenotype Analysis Step and an Index Calculation Step for Chromosomal Copy Number Abnormality Evaluation are performed (Example 7).

A form of analyzing a set of targets of analysis that satisfies the conditions (ii) and (iii):

- Embodiment in which a Parentage Testing Step and an Index Calculation Step for Chromosomal Copy Number Abnormality Evaluation are performed (Example 8).

Hereinafter, Examples 5 to 8 that can be adopted when the child is postnatal will be described in detail.

<3-4> Example 5 (Combination of Phenotype Analysis Step, Parentage Testing Step, and Index Calculation Step for Chromosomal Copy Number Abnormality Evaluation in Postnatal)

Example 5 is an embodiment including three types of test steps: a Parentage Testing Step, an Index Calculation Step for Chromosomal Copy Number Abnormality Evaluation, and a Phenotype Analysis Step, in addition to the genotyping step.
In Example 5, the set of targets of analysis that satisfies all the conditions (i) to (iii) is analyzed.

(i) The ratio of the number of single nucleotide polymorphism loci, which is known to have a phenotype to the total number of single nucleotide polymorphism loci in the set of targets of analysis is 10% or more.
(ii) The ratio of the number of single nucleotide polymorphism loci with a minor allele frequency of 1% to 50% and separated from each other by a distance of 20 kb or more on a chromosome to the total number of single nucleotide polymorphism loci in the set of targets of analysis is 10% or more.
(iii) The ratio of the number of single nucleotide polymorphism loci present on chromosome 13, chromosome 18, chromosome 21, chromosome X, and chromosome Y to the total number of single nucleotide polymorphism loci in the set of targets of analysis is 10% or more.

By analyzing a set of targets of analysis that satisfies all the conditions (i) to (iii), it is possible to obtain data that can be used for any of the three types of test steps: the Phenotype Analysis Step, the Parentage Testing Step, and the Index Calculation Step for Chromosomal Copy Number Abnormality Evaluation.
Next, for each single nucleotide polymorphism locus in the set of targets of analysis, a genotyping step of analyzing at least a paternal nucleic acid sample and a child's nucleic acid sample containing nucleic acids including the genetic information of the putative father is performed.
In the genotyping step, the genotyping of (a) to (c) described above can be performed. The following are specific embodiments and their descriptions:

- Embodiment in which genotyping of (a) putative father and (c) child is performed:
  PI calculation based on Table 5 can be performed in the Parentage Testing Step.

- Embodiment in which genotyping of (a) putative father, (b) mother, and (c) child is performed:
  PI calculation based on Table 6 can be performed in the Parentage Testing Step.

- Embodiment in which genotyping of (b) putative mother and (c) child is performed:
  MI calculation based on Table 7 can be performed in the Parentage Testing Step.

- Embodiment in which genotyping of (b) putative mother, (a) father, and (c) child is performed:
  MI calculation based on Table 8 can be performed in the Parentage Testing Step.

In Example 5, based on the result of the genotyping step, a Parentage Testing Step to determine the presence or absence of a parent-child relationship between the putative father and the child, or between the putative mother and the child, and a Phenotype Analysis Step are performed.

Further, based on the data obtained by analyzing the nucleic acid sample, an Index Calculation Step for Chromosomal Copy Number Abnormality Evaluation is performed.

<3-5> Example 6 (Combination of Parentage Testing Step and Phenotype Analysis Step in Postnatal)

Example 6 is an embodiment including a Parentage Testing Step and a Phenotype Analysis Step. By performing parentage testing, it is possible to confirm whether the minor allele is derived from the father or the mother.

In Example 6, a set of targets of analysis including single nucleotide polymorphism loci that satisfy the following conditions (i) and (ii) is analyzed.

(i) The ratio of the number of single nucleotide polymorphism loci, which is known to have a phenotype to the total number of single nucleotide polymorphism loci in the set of targets of analysis is 10% or more.

(ii) The ratio of the number of single nucleotide polymorphism loci with a minor allele frequency of 1% to 50% and separated from each other by a distance of 20 kb or more on a chromosome to the total number of single nucleotide polymorphism loci in the set of targets of analysis is 10% or more.

Regarding the embodiment of genotyping performed on the set of targets of analysis that satisfies the above conditions, the description of Example 5 can be applied as it is.

In Example 6, by analyzing a set of targets of analysis that satisfies conditions (i) and (ii), data usable for the Phenotype Analysis Step and Parentage Testing Step can be obtained.

In Example 6, based on the result of the genotyping step, a Parentage Testing Step of determining the presence or absence of a parent-child relationship between the putative father and the child or the presence or absence of a parent-child relationship between the putative mother and the child, and a Phenotype Analysis Step are performed.

In Examples 5 and 6, in the Parentage Testing Step, when the parent-child relationship between the putative father and the child or the parent-child relationship between the putative mother and the child is affirmed, the Phenotype Analysis Step may be performed. Conversely, if the parent-child relationship is denied, the Phenotype Analysis Step may not be performed.

It should be noted that the Phenotype Analysis Step can be performed regardless of the result of the Parentage Testing Step.

In Examples 5 and 6, parentage testing and phenotype analysis can be performed based on data obtained by a single sample analysis. In the present embodiment, by confirming the parent-child relationship between the putative father and the child or the putative mother and the child in the Parentage Testing Step, it becomes possible to determine which risk allele derived from the father or the mother is inherited by the child in the Phenotype Analysis Step.

<3-6> Example 7 (Combination of Phenotype Analysis Step and Index Calculation Step for Chromosomal Copy Number Abnormality Evaluation in Postnatal)

Example 7 is an embodiment including both an Index Calculation Step for Chromosomal Copy Number Abnormality Evaluation and a Phenotype Analysis Step in addition to the genotyping step. According to Example 7, based on data obtained by a single sample analysis, it is possible to perform two types of test steps, an Index Calculation Step for Chromosomal Copy Number Abnormality Evaluation and a Phenotype Analysis Step, at once, which is an advantageous effect.

In Example 7, a set of targets of analysis including single nucleotide polymorphism loci that satisfy the following conditions (i) and (iii) is analyzed.

(i) The ratio of the number of single nucleotide polymorphism loci, which is known to have a phenotype to the total number of single nucleotide polymorphism loci included in the set of targets of analysis is 10% or more.

(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

By analyzing a set of targets of analysis including single nucleotide polymorphism loci that satisfy the conditions (i) and (iii), it is possible to acquire data that can be used for both the Phenotype Analysis Step and the Index Calculation Step for Chromosomal Copy Number Abnormality Evaluation. This allows Example 7 to perform both the Index Calculation Step for Chromosomal Copy Number Abnormality Evaluation and the Phenotype Analysis Step.

In Example 7, genotyping is performed using the genomic DNA of the test subject, and phenotype analysis is performed.

The specific embodiments of the Index Calculation Step for Chromosomal Copy Number Abnormality Evaluation and the Phenotype Analysis Step in Example 7 can be applied as described above.

<3-7> Example 8 (Combination of Parentage Testing Step and Index Calculation Step for Chromosomal Copy Number Abnormality Evaluation in Postnatal)

Example 8 is an embodiment including a Parentage Testing Step and an Index Calculation Step for Chromosomal Copy Number Abnormality Evaluation in addition to the genotyping step. According to Example 8, parentage testing and evaluation of chromosomal numerical abnormalities can be performed based on data obtained by a single sample analysis.

In Example 8, a set of targets of analysis including single nucleotide polymorphism loci that satisfy the following conditions (ii) and (iii) is analyzed.

(ii) The ratio of the number of single nucleotide polymorphism loci with a minor allele frequency of 1% to 50% and separated from each other by a distance of 20 kb or more on a chromosome to the total number of single nucleotide polymorphism loci included in the set of targets of analysis is 10% or more.

(iii) The ratio of the number of single nucleotide polymorphism loci present on chromosome 13, chromosome 18, chromosome 21, chromosome X, and chromosome Y to the total number of single nucleotide polymorphism loci included in the set of targets of analysis is 10% or more.

By analyzing the set of targets of analysis, data that can be used for both the Parentage Testing Step and the Index Calculation Step for Chromosomal Copy Number Abnormality Evaluation can be obtained. This allows Example 8 to perform both the Parentage Testing Step and the Index Calculation Step for Chromosomal Copy Number Abnormality Evaluation.

The description of Example 5 can be directly applied to the embodiment of the genotyping step in Example 8. In addition, the above description can be directly applied to the specific embodiments of the Parentage Testing Step and the Index Calculation Step for Chromosomal Copy Number Abnormality Evaluation in Example 8.

Example

<Test Case 1> CPI Simulation

A simulation was conducted to evaluate the effect of the minor allele frequency in SNVs constituting the set of targets of analysis, which is a set of SNVs used for parentage testing, on CPI. In this test case, it is assumed that parentage testing is performed based solely on the genotyping results of the putative father and the child.

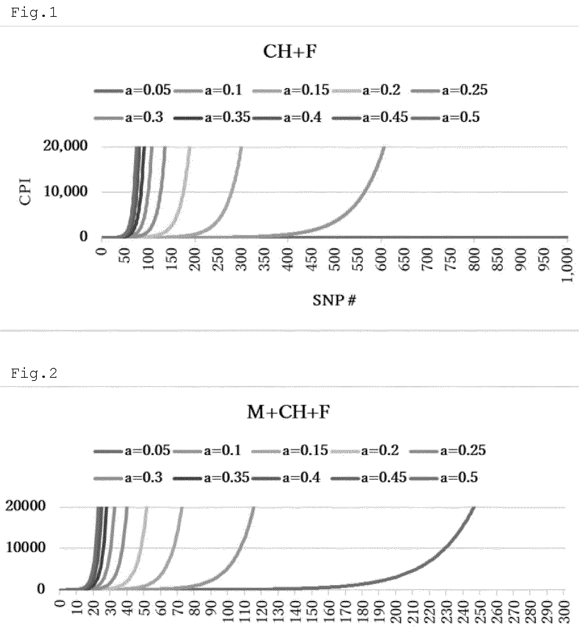

Genotyping was performed using an set of targets of analysis composed of SNVs with a minor allele frequency of a single value ranging from 0.05 to 0.5. It was assumed that all SNVs in the set of targets of analysis exhibited a genotype consistent with the putative father being the biological father of the child. The calculated CPI values were plotted on the vertical axis, while the number of SNVs included in the set of targets of analysis was plotted on the horizontal axis (Figure 1).

As shown in Figure 1, when using an set of targets of analysis composed exclusively of SNVs with a minor allele frequency of 0.05, even when the number of SNVs exceeded 1,000, an effective CPI sufficient to affirm the parent-child relationship could not be obtained (Figure 1).

On the other hand, when using an set of targets of analysis composed solely of SNVs with a minor allele frequency of 0.1 or higher, it was possible to calculate a CPI value sufficient to affirm the parent-child relationship. Furthermore, it was demonstrated that as the minor allele frequency increased, the CPI reached 10,000 with a smaller number of SNVs.

<Test Case 2> CPI Simulation

As in Test Case 1, a simulation was performed to evaluate the effect of minor allele frequency in SNVs constituting the set of targets of analysis used for parentage testing on CPI. In this test case, it is assumed that parentage testing is performed based on the genotyping results of the mother, putative father, and child.

Genotyping was conducted using an set of targets of analysis composed of SNVs in which the occurrence frequency of minor alleles had a single value ranging from 0.05 to 0.5. It was assumed that all SNVs in the set of targets of analysis exhibited genotypes consistent with the putative father being the child's biological father. The calculated CPI values were plotted on the vertical axis, while the number of SNVs included in the set of targets of analysis was plotted on the horizontal axis (Figure 2).

As shown in Figure 2, even when using an set of targets of analysis composed solely of SNVs with a minor allele frequency of 0.05, a CPI sufficient to affirm the parent-child relationship was obtained (Figure 2). Furthermore, it was demonstrated that as the minor allele frequency increased, a CPI of 10,000 was reached with a smaller number of SNVs (Figure 2).

Industrial Applications

The present invention can be applied to parentage testing, chromosomal numerical abnormality testing, and the analysis of DTC testing or single-gene disorders.

Table 9-1

[Table 9-1]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 1 | rs1022580 | 1 | 17359676 | C | A | 0.041 | 0.959 | C | 0.041 | Medial epicondylitis | - |
| 2 | rs2476601 | 1 | 114377568 | A | G | 0.027 | 0.973 | A | 0.027 | Hodgkin's lymphoma | 4.0E-07 |
| 3 | rs6339 | 1 | 156848946 | G | T | 0.977 | 0.023 | T | 0.023 | Lymphocyte counts | 3.0E-11 |
| 4 | rs3730238 | 1 | 201330429 | T | C | 0.903 | 0.097 | C | 0.097 | Cardiomyopathy,Hypertrophic cardiomyopathy,Left ventricular noncompaction cardiomyopathy, Primary familial hypertrophic cardiomyopathy,Familial restrictive cardiomyopathy,Cardiovascular phenotype,Dilated Cardiomyopathy, | 3.6E-03 |
| 5 | rs1805087 | 1 | 237048500 | A | G | 0.782 | 0.218 | G | 0.218 | Neural tube defects, folate-sensitiv,Gastrointestinal stroma tumor,Disorders of Intracellular Cobalamin Metabolism, | - |
| 6 | rs1260326 | 2 | 27730940 | T | C | 0.293 | 0.707 | T | 0.293 | Total triglycerides levels | - |
| 7 | rs3791675 | 2 | 56111309 | C | T | 0.709 | 0.291 | T | 0.291 | Height | 3.0E-07 |
| 8 | rs11684404 | 2 | 88924622 | T | C | 0.660 | 0.340 | C | 0.340 | Brainstem volume | 4.0E-09 |
| 9 | rs13015714 | 2 | 102971865 | G | T | 0.308 | 0.692 | G | 0.308 | Extranodal natural killer T-cell lymphoma (nasal type) | 2.8E-16 |
| 10 | rs62158211 | 2 | 114106139 | G | T | 0.848 | 0.152 | T | 0.152 | Sleep duration (oversleepers vs undersleepers) | 1.0E-07 |
| 11 | rs7570971 | 2 | 135837906 | C | A | 0.157 | 0.843 | C | 0.157 | Gut microbiota (bacterial taxa, rank normal transformation method) | 1.0E-06 |
| 12 | rs16866933 | 2 | 180566678 | G | A | 0.839 | 0.161 | A | 0.161 | Sudden cardiac arrest | 6.0E-14 |
| 13 | rs5742926 | 2 | 190648805 | G | T | 0.912 | 0.088 | T | 0.088 | Lynch syndrome (colon cancer) | - |
| 14 | rs5031011 | 2 | 215632192 | G | A | 0.648 | 0.352 | A | 0.352 | Neoplasm of the breast,Hereditary cancer-predisposing syndrome, | 8.4E-06 |

33

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 15 | rs4321351 | 2 | 230129493 | A | G | 0.820 | 0.180 | G | 0.180 | HDL cholesterol | 7.5E-05 |
| 16 | rs34290285 | 2 | 242698640 | G | A | 0.755 | 0.245 | A | 0.245 | Asthma onset (childhood vs adult) | 2.0E-08 |
| 17 | rs2340917 | 3 | 14175262 | T | C | 0.537 | 0.463 | C | 0.463 | Cardiomyopathy,Arrhythmogenic right ventricular cardiomyopathy,Arrhythmogenic right ventricular cardiomyopathy | - |
| 18 | rs1482853 | 3 | 156798473 | C | A | 0.655 | 0.345 | A | 0.345 | Anthropometric traits in newborns | 4.0E-09 |
| 19 | rs2276792 | 3 | 189582249 | G | A | 0.780 | 0.220 | A | 0.220 | iron deficiency anaemia | - |
| 20 | rs789859 | 3 | 194405888 | G | T | 0.612 | 0.388 | T | 0.388 | Non-lobar intracerebral hemorrhage (MTAG) | 1.0E-06 |

Table 9-2

[Table 9-2]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 21 | rs6449353 | 4 | 18033488 | T | C | 0.731 | 0.269 | C | 0.269 | Birth length | 1.0E-06 |
| 22 | rs8192678 | 4 | 23815662 | C | T | 0.734 | 0.266 | T | 0.266 | Blood pressure | - |
| 23 | rs2199936 | 4 | 89045331 | A | G | 0.150 | 0.850 | A | 0.150 | Lipoprotein-associated phospholipase A2 activity change in response to statin therapy | 2.0E-10 |
| 24 | rs2200733 | 4 | 111710169 | C | T | 0.753 | 0.247 | T | 0.247 | Stroke (ischemic) | 2.0E-10 |
| 25 | rs4148254 | 4 | 115544713 | C | T | 0.950 | 0.050 | T | 0.050 | Red blood cell (erythrocyte) count | 4.6E-06 |
| 26 | rs8396 | 4 | 159630817 | T | C | 0.737 | 0.263 | C | 0.263 | Blood protein levels | 5.0E-86 |
| 27 | rs1806729 | 4 | 169432841 | G | A | 0.764 | 0.236 | A | 0.236 | Carcinoma of pancreas, | 7.5E-05 |
| 28 | rs1801394 | 5 | 7870973 | A | G | 0.636 | 0.364 | G | 0.364 | Down syndrome, Neural tube defects, folatesensitive, Gastrointestinal stroma tumor, Disorders of Intracellular Cobalamin Metabolism, methotrexate response - Toxicity/ADR | 1.0E-02 |
| 29 | rs112724034 | 5 | 109221026 | C | T | 0.936 | 0.064 | T | 0.064 | Alzheimer's disease (cognitive decline) | 9.0E-13 |
| 30 | rs1042713 | 5 | 148206440 | G | A | 0.524 | 0.476 | A | 0.476 | salbutamol response - Efficacy, salmeterol response - Efficacy | 3.0E-03 |
| 31 | rs6882776 | 5 | 172664163 | G | A | 0.577 | 0.423 | A | 0.423 | Heart rate | 2.0E-12 |

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 32 | rs2076299 | 6 | 7580958 | A | G | 0.797 | 0.203 | G | 0.203 | Cardiomyopathy,Arrhythmogenic right ventricular cardiomyopathy,Skin fra-gility woolly hair syndro-me,Ectodermal dysplasia skin fragility syndrome,Epidermolysis bullosa, I | 1.7E-03 |
| 33 | rs130067 | 6 | 31118511 | T | G | 0.745 | 0.255 | G | 0.255 | Prostate cancer | 2.0E-07 |
| 34 | rs2307456 | 6 | 43565568 | G | T | 0.996 | 0.004 | T | 0.004 | Xeroderma pigmentosum, variant type, | - |
| 35 | rs7759938 | 6 | 105378954 | C | T | 0.385 | 0.615 | C | 0.385 | Waist circumference adjusted for BMI in inactive individuals | 7.0E-07 |
| 36 | rs4273712 | 6 | 126964510 | A | G | 0.715 | 0.285 | G | 0.285 | Type 2 diabetes | 3.0E-12 |
| 37 | rs4869742 | 6 | 151907748 | C | T | 0.428 | 0.572 | C | 0.428 | Ultradistal forearm bone mineral density | 2.0E-19 |
| 38 | rs2234693 | 6 | 152163335 | T | C | 0.554 | 0.446 | C | 0.446 | Heel bone mineral density | 9.0E-03 |
| 39 | rs10455872 | 6 | 161010118 | A | G | 0.978 | 0.022 | G | 0.022 | Parental longevity (father's age at death) | 5.0E-07 |
| 40 | rs1522280 | 7 | 22368678 | T | C | 0.707 | 0.293 | C | 0.293 | face recognition | 9.7E-08 |
| 41 | rs12534093 | 7 | 23502974 | T | A | 0.834 | 0.166 | A | 0.166 | Infant length | 8.0E-08 |

Table 9-3

[Table 9-3]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 42 | rs1128503 | 7 | 87179601 | A | G | 0.416 | 0.584 | A | 0.416 | noninflammatory Crohn's disease | 4.7E-02 |
| 43 | rs17477177 | 7 | 106411858 | T | C | 0.832 | 0.168 | C | 0.168 | Pulse pressure | 1.0E-09 |
| 44 | rs7776725 | 7 | 121033121 | T | C | 0.754 | 0.246 | C | 0.246 | Bone mineral density (femoral neck) | 1.0E-06 |
| 45 | rs713598 | 7 | 141673345 | C | G | 0.495 | 0.505 | C | 0.495 | Tea consumption | 3.0E-10 |
| 46 | rs9987289 | 8 | 9183358 | A | G | 0.114 | 0.886 | A | 0.114 | LDL cholesterol levels | 2.0E-36 |
| 47 | rs56233017 | 8 | 144981488 | G | A | 0.988 | 0.012 | A | 0.012 | Diastolic blood pressure | 5.0E-12 |
| 48 | rs11144688 | 9 | 78542286 | G | A | 0.941 | 0.059 | A | 0.059 | Waist circumference adjusted for BMI in non-smokers | 1.0E-06 |
| 49 | rs3176752 | 9 | 100437487 | G | T | 0.939 | 0.061 | T | 0.061 | neuroblastoma | 4.9E-02 |
| 50 | rs1805313 | 9 | 116151191 | A | G | 0.547 | 0.453 | G | 0.453 | Hemoglobin (Hb) content | 4.0E-14 |
| 51 | rs4962081 | 9 | 135772717 | G | A | 0.938 | 0.062 | A | 0.062 | Bladder cancer, somatic,Hereditary cancer-predisposing syndrome,Tuberous sclerosis syndrome,Lymphangiomyomatosis,Focal cortical dysplasia type II,Tuberous sclerosis 1 | - |
| 52 | rs17135159 | 10 | 3408947 | A | C | 0.745 | 0.255 | C | 0.255 | Reading and spelling | 2.4E-06 |
| 53 | rs3905706 | 10 | 28479942 | C | T | 0.610 | 0.390 | T | 0.390 | Lumbar spine bone mineral density | 2.0E-16 |
| 54 | rs1171614 | 10 | 61469538 | T | C | 0.159 | 0.841 | T | 0.159 | Hemoglobin concentration | 1.0E-10 |

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 55 | rs28371685 | 10 | 96740981 | C | T | 0.993 | 0.007 | T | 0.007 | Lesinurad response,Flurbiprofen response,Piroxicam response, | - |
| 56 | rs7080536 | 10 | 115348046 | G | A | 0.992 | 0.008 | A | 0.008 | Eosinophil counts | 7.0E-09 |
| 57 | rs3858340 | 10 | 121436286 | C | T | 0.844 | 0.156 | T | 0.156 | Dilated cardiomyopathy | 3.1E-12 |
| 58 | rs755793 | 10 | 123310871 | A | G | 0.874 | 0.126 | G | 0.126 | Crouzon syndrome,FGFR2 related craniosynostosis, | - |
| 59 | rs2030323 | 11 | 27728539 | A | C | 0.240 | 0.760 | A | 0.240 | Body mass index | 4.0E-16 |
| 60 | rs1815739 | 11 | 66328095 | T | C | 0.401 | 0.599 | T | 0.401 | muscle performance | 8.0E-12 |
| 61 | rs3736228 | 11 | 68201295 | C | T | 0.884 | 0.116 | T | 0.116 | Appendicular lean mass | 1.0E-09 |
| 62 | rs964184 | 11 | 116648917 | G | C | 0.222 | 0.778 | G | 0.222 | hypertriglyceridemia | 5.0E-24 |
| 63 | rs75190942 | 11 | 128764571 | C | A | 0.922 | 0.078 | A | 0.078 | Atrial fibrillation | 3.0E-08 |
| 64 | rs3764030 | 12 | 14133332 | C | T | 0.759 | 0.241 | T | 0.241 | mental processing speed over aging | 1.0E-03 |
| 65 | rs1046116 | 12 | 33021934 | A | G | 0.849 | 0.151 | G | 0.151 | Arrhythmogenic right ventricular cardiomyopathy | 9.0E-04 |
| 66 | rs10784502 | 12 | 66343810 | C | T | 0.341 | 0.659 | C | 0.341 | Appendicular lean mass | 7.0E-12 |
| 67 | rs11105364 | 12 | 90069276 | T | G | 0.799 | 0.201 | G | 0.201 | Hypertension | 1.0E-06 |

Table 9-4

[Table 9-4]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 68 | rs671 | 12 | 112241766 | G | A | 0.964 | 0.036 | A | 0.036 | Sweet taste preference | 2.8E-70 |
| 69 | rs5745068 | 12 | 133202740 | C | T | 0.990 | 0.010 | T | 0.010 | Hereditary cancer-pre-disposing syndrome,Co-lorectal cancer | - |
| 70 | rs60610174 | 13 | 20469353 | G | A | 0.898 | 0.102 | A | 0.102 | Alcohol use disorder (consumption score) | 3.0E-08 |
| 71 | rs9552911 | 13 | 23864657 | G | A | 0.917 | 0.083 | A | 0.083 | Type 2 diabetes | 2.0E-08 |
| 72 | rs753955 | 13 | 24293859 | A | G | 0.556 | 0.444 | G | 0.444 | Lung cancer | 2.0E-12 |
| 73 | rs76080105 | 13 | 28599094 | T | C | 0.995 | 0.005 | C | 0.005 | Monocyte count | 2.0E-16 |
| 74 | rs151330264 | 13 | 32988865 | A | T | 0.992 | 0.008 | T | 0.008 | Total cholesterol levels | 1.0E-09 |
| 75 | rs9603616 | 13 | 40368069 | C | T | 0.734 | 0.266 | T | 0.266 | Rheumatoid arthritis | 8.0E-11 |
| 76 | rs9533090 | 13 | 42951449 | C | T | 0.716 | 0.284 | T | 0.284 | Bone mineral density | 5.0E-25 |
| 77 | rs8001611 | 13 | 42965694 | C | T | 0.311 | 0.689 | C | 0.311 | Total body bone mineral density (age 30-45) | 2.0E-10 |
| 78 | rs9533095 | 13 | 42969049 | G | T | 0.714 | 0.286 | T | 0.286 | Serum alkaline phosphatase levels | 8.0E-10 |
| 79 | rs9594759 | 13 | 43032593 | C | T | 0.557 | 0.443 | T | 0.443 | Bone mineral density (spine) | 2.0E-17 |
| 80 | rs1021188 | 13 | 43116133 | C | T | 0.224 | 0.776 | C | 0.224 | Bone mineral density | 4.0E-14 |
| 81 | rs79927049 | 13 | 43331787 | A | T | 0.995 | 0.005 | T | 0.005 | Total body bone mineral density | 2.0E-08 |
| 82 | rs9526023 | 13 | 45524626 | G | A | 0.964 | 0.036 | A | 0.036 | HDL cholesterol levels | 4.0E-09 |
| 83 | rs3092904 | 13 | 49051481 | T | A | 0.811 | 0.189 | A | 0.189 | Retinoblastoma, | - |
| 84 | rs2687950 | 13 | 50718468 | C | T | 0.837 | 0.163 | T | 0.163 | Height | 9.0E-27 |
| 85 | rs3118906 | 13 | 51106788 | G | A | 0.870 | 0.130 | A | 0.130 | Height | 2.0E-24 |

42

(continued)

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 86 | rs3116613 | 13 | 51143055 | T | G | 0.914 | 0.086 | G | 0.086 | Urinary albumin-to-creatinine ratio | 4.0E-08 |
| 87 | rs7994724 | 13 | 51445560 | A | G | 0.199 | 0.801 | A | 0.199 | Haemorrhoidal disease | 2.0E-17 |
| 88 | rs6561599 | 13 | 51478918 | C | G | 0.466 | 0.534 | C | 0.466 | Benign prostatic hyperplasia and lower urinary tract symptoms | 1.0E-08 |
| 89 | rs2806949 | 13 | 53646138 | A | G | 0.326 | 0.674 | A | 0.326 | Trans fatty acid levels | 1.0E-06 |
| 90 | rs1443914 | 13 | 53917230 | T | C | 0.470 | 0.530 | T | 0.470 | Multisite chronic pain | 3.0E-11 |
| 91 | rs9316619 | 13 | 53978628 | T | C | 0.933 | 0.067 | C | 0.067 | Insomnia | 6.0E-09 |
| 92 | rs2801617 | 13 | 56470841 | T | C | 0.979 | 0.021 | C | 0.021 | Left ventricular end-diastolic volume (MTAG) | 1.0E-08 |
| 93 | rs116967397 | 13 | 58466434 | A | C | 0.997 | 0.003 | C | 0.003 | Educational attainment (MTAG) | 3.0E-08 |
| 94 | rs111839202 | 13 | 59594990 | A | T | 0.984 | 0.016 | T | 0.016 | Self-reported math ability | 5.0E-08 |

Table 9-5

[Table 9-5]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 95 | rs76065946 | 13 | 60373704 | A | T | 0.944 | 0.056 | T | 0.056 | Breast milk fatty acid composition (infant genotype effect) | 5.0E-07 |
| 96 | rs9528384 | 13 | 62302565 | A | G | 0.818 | 0.182 | G | 0.182 | Verbal declarative memory | 1.0E-06 |
| 97 | rs9540128 | 13 | 65078467 | C | T | 0.717 | 0.283 | T | 0.283 | Takayasu arteritis | 1.0E-07 |
| 98 | rs1953124 | 13 | 66346047 | C | G | 0.182 | 0.818 | C | 0.182 | Liver fibrosis in non-alcoholic fatty liver disease | 4.0E-07 |
| 99 | rs138607199 | 13 | 68268012 | G | A | 0.996 | 0.004 | A | 0.004 | Post bronchodilator FEV1/FVC ratio | 4.0E-07 |
| 100 | rs141008343 | 13 | 68309045 | T | A | 0.996 | 0.004 | A | 0.004 | Post bronchodilator FEV1/FVC ratio | 4.0E-07 |
| 101 | rs9529561 | 13 | 69899506 | A | G | 0.964 | 0.036 | G | 0.036 | Mortality in sepsis | 3.0E-07 |
| 102 | rs9542155 | 13 | 70579527 | T | C | 0.373 | 0.627 | T | 0.373 | Tonsillitis | 2.0E-11 |
| 103 | rs3861113 | 13 | 72364382 | C | A | 0.637 | 0.363 | A | 0.363 | Diastolic blood pressure | 4.0E-11 |
| 104 | rs340561 | 13 | 72848156 | G | T | 0.776 | 0.224 | T | 0.224 | Restless legs syndrome | 3.0E-09 |
| 105 | rs9573092 | 13 | 73627275 | A | G | 0.565 | 0.435 | G | 0.435 | Allergic disease (asthma, hay fever or eczema) | 5.6E-12 |
| 106 | rs112679104 | 13 | 74706951 | C | T | 0.994 | 0.006 | T | 0.006 | Low density lipoprotein cholesterol levels | 6.0E-10 |
| 107 | rs12429889 | 13 | 74742322 | T | C | 0.685 | 0.315 | C | 0.315 | Sudden cardiac arrest | 5.0E-20 |
| 108 | rs9565170 | 13 | 76064659 | G | C | 0.891 | 0.109 | C | 0.109 | Mean corpuscular volume | 1.0E-11 |
| 109 | rs9573999 | 13 | 77696256 | C | T | 0.934 | 0.066 | T | 0.066 | Morning person | 1.0E-18 |
| 110 | rs9602270 | 13 | 84281063 | A | T | 0.897 | 0.103 | T | 0.103 | Squamous cell lung carcinoma | 3.0E-07 |

45

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 111 | rs112108364 | 13 | 86490590 | T | G | 0.860 | 0.140 | G | 0.140 | Predicted visceral adipose tissue | 3.0E-10 |
| 112 | rs7323893 | 13 | 88704706 | T | C | 0.971 | 0.029 | C | 0.029 | HDL cholesterol | 1.0E-07 |
| 113 | rs80092143 | 13 | 91057759 | C | G | 0.869 | 0.131 | G | 0.131 | Gait rhythm | 7.0E-07 |
| 114 | rs16946160 | 13 | 92203813 | G | A | 0.837 | 0.163 | A | 0.163 | Nephrotic syndrome (acquired) | 3.0E-07 |
| 115 | rs72635657 | 13 | 93898697 | G | A | 0.997 | 0.003 | A | 0.003 | Heel bone mineral density | 6.0E-09 |
| 116 | rs9561329 | 13 | 94011169 | A | G | 0.796 | 0.204 | G | 0.204 | Neuroticism (age interaction) | 1.0E-07 |
| 117 | rs61965473 | 13 | 95571786 | C | T | 0.993 | 0.007 | T | 0.007 | Lymphocytic leukemia | 4.0E-08 |
| 118 | rs61967710 | 13 | 97176585 | G | A | 0.961 | 0.039 | A | 0.039 | Type 2 diabetes | 3.0E-08 |
| 119 | rs1564454 | 13 | 98943639 | A | G | 0.637 | 0.363 | G | 0.363 | Ischemic stroke (large artery atherosclerosis) | 1.0E-07 |
| 120 | rs2153672 | 13 | 100300942 | C | T | 0.837 | 0.163 | T | 0.163 | Heel bone mineral density | 3.0E-13 |
| 121 | rs3783177 | 13 | 101151360 | T | G | 0.873 | 0.127 | G | 0.127 | Smoking status (ever vs never smokers) | 2.0E-09 |
| 122 | rs751402 | 13 | 103498198 | A | G | 0.311 | 0.689 | A | 0.311 | Xeroderma pigmentosum | 8.0E-03 |
| 123 | rs16961023 | 13 | 103663945 | C | G | 0.962 | 0.038 | G | 0.038 | Late-onset Alzheimer's disease | 5.0E-08 |

Table 9-6

[Table 9-6]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 124 | rs9558301 | 13 | 105144448 | C | T | 0.444 | 0.556 | C | 0.444 | Smoking initiation (ever regular vs never regular) (MTAG) | 2.0E-08 |
| 125 | rs75065922 | 13 | 105811198 | G | A | 0.998 | 0.002 | A | 0.002 | Ischemic stroke in diabetes mellitus | 4.0E-07 |
| 126 | rs61967180 | 13 | 107995343 | A | G | 0.980 | 0.020 | G | 0.020 | Height | 2.0E-07 |
| 127 | rs374039502 | 13 | 108960385 | T | A | 0.987 | 0.013 | A | 0.013 | Apolipoprotein A1 levels | 1.0E-09 |
| 128 | rs912322 | 13 | 109808286 | A | G | 0.746 | 0.254 | G | 0.254 | Alzheimer's disease | 1.0E-06 |
| 129 | rs9583531 | 13 | 111385161 | T | G | 0.861 | 0.139 | G | 0.139 | Coronary artery disease | 5.0E-08 |
| 130 | rs11842874 | 13 | 113694509 | A | G | 0.868 | 0.132 | G | 0.132 | Osteoarthritis | 2.0E-08 |
| 131 | rs365990 | 14 | 23861811 | A | G | 0.625 | 0.375 | G | 0.375 | Electrocardiogram morphology (amplitude at temporal datapoints) | 4.0E-08 |
| 132 | rs4556 | 14 | 75476071 | G | C | 0.482 | 0.518 | G | 0.482 | Coronary artery disease | 9.6E-09 |
| 133 | rs1991517 | 14 | 81610583 | G | C | 0.103 | 0.897 | G | 0.103 | Congenital hypothyroidism | - |
| 134 | rs2073333 | 14 | 94844562 | C | T | 0.721 | 0.279 | T | 0.279 | Serum levels of protein Glycogen synthase kinase 3 | 6.0E-302 |
| 135 | rs12913832 | 15 | 28365618 | A | G | 0.823 | 0.177 | G | 0.177 | Intraocular pressure | 9.0E-07 |
| 136 | rs1045204 | 15 | 34634138 | G | A | 0.795 | 0.205 | A | 0.205 | Dyskeratosis Congenita, Recessive | - |
| 137 | rs12912233 | 15 | 61267096 | C | T | 0.666 | 0.334 | T | 0.334 | Depression (quantitative trait) | 6.0E-07 |
| 138 | rs1051730 | 15 | 78894339 | G | A | 0.832 | 0.168 | A | 0.168 | Smoking behaviour (Nicotine dependence) | 6.0E-20 |

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 139 | rs16942341 | 15 | 89388905 | C | T | 0.938 | 0.063 | T | 0.063 | Hip circumference adjusted for BMI | 4.0E-08 |
| 140 | rs28384991 | 15 | 91295110 | C | T | 0.977 | 0.023 | T | 0.023 | Bloom syndrome,Hereditary cancer-predisposing syndrome, | - |
| 141 | rs2871865 | 15 | 99194896 | C | G | 0.775 | 0.225 | G | 0.225 | Height | 6.0E-07 |
| 142 | rs1799801 | 16 | 14041958 | T | C | 0.759 | 0.241 | C | 0.241 | Xeroderma pigmentosum, | - |
| 143 | rs8053188 | 16 | 23652525 | C | T | 0.934 | 0.066 | T | 0.066 | Fanconi anemia,Hereditary cancer-predisposing syndrome, | - |
| 144 | rs2066852 | 16 | 50827518 | C | T | 0.921 | 0.079 | T | 0.079 | Cylindromatosis, familial,Spiegler-Brooke syndrome,Trichoepithelioma multiple familial 2 | - |
| 145 | rs8050136 | 16 | 53816275 | C | A | 0.678 | 0.322 | A | 0.322 | Body mass index | 1.0E-07 |
| 146 | rs10048146 | 16 | 86710660 | A | G | 0.839 | 0.161 | G | 0.161 | Bone mineral density (hip) | 2.0E-07 |
| 147 | rs28363283 | 17 | 33430316 | C | T | 0.995 | 0.005 | T | 0.005 | Ovarian cancer | 4.8E-06 |
| 148 | rs1060915 | 17 | 41234470 | A | G | 0.664 | 0.336 | G | 0.336 | breast and ovarian cancer syndrome | - |

EP 4 582 539 A1

Table 9-7

[Table 9-7]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 149 | rs9900627 | 17 | 46805443 | A | G | 0.879 | 0.121 | G | 0.121 | Prostate Cancer Risk | 1.0E-03 |
| 150 | rs7221274 | 17 | 57008128 | A | G | 0.633 | 0.367 | G | 0.367 | Testicular cancer | 4.0E-09 |
| 151 | rs2240307 | 17 | 63554307 | A | G | 0.921 | 0.079 | G | 0.079 | Lung cancer | 7.4E-24 |
| 152 | rs77542162 | 17 | 67081278 | A | G | 0.996 | 0.004 | G | 0.004 | Total cholesterol levels | 2.0E-41 |
| 153 | rs9948462 | 18 | 7076836 | C | T | 0.445 | 0.555 | C | 0.445 | Type 2 diabetes | 9.0E-10 |
| 154 | rs113776891 | 18 | 7754654 | T | C | 0.982 | 0.018 | C | 0.018 | Recurrence of mild malaria attacks | 1.0E-06 |
| 155 | rs11872481 | 18 | 9128453 | G | A | 0.925 | 0.075 | A | 0.075 | Red cell distribution width | 6.0E-13 |
| 156 | rs28926173 | 18 | 13886719 | G | A | 0.986 | 0.014 | A | 0.014 | Parental longevity (both parents in top 10%) | 2.0E-08 |
| 157 | rs11872104 | 18 | 14265825 | A | G | 0.556 | 0.444 | G | 0.444 | Myopia (age of diagnosis) | 8.0E-15 |
| 158 | rs118035855 | 18 | 18994683 | G | A | 0.995 | 0.005 | A | 0.005 | Reticulocyte count | 2.0E-09 |
| 159 | rs9949617 | 18 | 20879217 | C | T | 0.762 | 0.238 | T | 0.238 | Hypertriglyceridemia | 2.0E-08 |
| 160 | rs10502437 | 18 | 20970706 | G | A | 0.696 | 0.304 | A | 0.304 | Osteoarthritis | 3.0E-08 |
| 161 | rs1805081 | 18 | 21140432 | T | C | 0.781 | 0.219 | C | 0.219 | Obesity | 3.0E-07 |
| 162 | rs9959491 | 18 | 25406855 | C | T | 0.465 | 0.535 | C | 0.465 | Breast cancer | 5.0E-07 |
| 163 | rs356833 | 18 | 26323525 | A | G | 0.709 | 0.291 | G | 0.291 | Systolic blood pressure | 1.0E-08 |
| 164 | rs11083411 | 18 | 28257199 | T | G | 0.651 | 0.349 | G | 0.349 | Metabolite levels | 9.0E-09 |
| 165 | rs57261374 | 18 | 28423906 | G | C | 0.956 | 0.044 | C | 0.044 | Type 2 diabetes | 1.0E-06 |
| 166 | rs117618124 | 18 | 29977689 | T | C | 0.983 | 0.017 | C | 0.017 | Breast cancer | 6.0E-12 |

EP 4 582 539 A1

(continued)

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 167 | rs10164193 | 18 | 31161426 | T | G | 0.883 | 0.117 | G | 0.117 | Diastolic blood pressure | 2.0E-11 |
| 168 | rs2303510 | 18 | 34324091 | G | A | 0.624 | 0.376 | A | 0.376 | Dilated cardiomyopathy (MTAG) | 4.0E-09 |
| 169 | rs7242036 | 18 | 35247852 | C | G | 0.472 | 0.528 | C | 0.472 | Depressive symptoms | 3.0E-15 |
| 170 | rs4799464 | 18 | 35278126 | C | T | 0.482 | 0.518 | C | 0.482 | Life satisfaction | 1.0E-10 |
| 171 | rs12961969 | 18 | 35364098 | A | C | 0.143 | 0.857 | A | 0.143 | Neuroticism | 9.0E-07 |
| 172 | rs62098997 | 18 | 37289059 | G | A | 0.975 | 0.025 | A | 0.025 | Highest math class taken | 4.0E-08 |
| 173 | rs989885 | 18 | 38464855 | A | G | 0.812 | 0.188 | G | 0.188 | Morningness | 4.0E-08 |
| 174 | rs16975921 | 18 | 39914870 | A | T | 0.684 | 0.316 | T | 0.316 | Body mass index | 1.0E-11 |
| 175 | rs6507583 | 18 | 42399590 | A | G | 0.831 | 0.169 | G | 0.169 | Breast cancer | 1.0E-06 |
| 176 | rs11876432 | 18 | 42669425 | C | G | 0.859 | 0.141 | G | 0.141 | Smoking status (ever vs never smokers) | 7.0E-09 |
| 177 | rs10502861 | 18 | 42800148 | C | T | 0.696 | 0.304 | T | 0.304 | Male-pattern baldness | 3.0E-09 |
| 178 | rs11082438 | 18 | 42865210 | G | T | 0.924 | 0.076 | T | 0.076 | Follicular lymphoma | 4.0E-07 |

Table 9-8

[Table 9-8]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 179 | rs2576037 | 18 | 44585420 | C | T | 0.555 | 0.445 | T | 0.445 | Conscientiousness | 1.0E-10 |
| 180 | rs8087252 | 18 | 46041755 | A | G | 0.216 | 0.784 | A | 0.216 | Rheumatoid arthritis | 7.0E-09 |
| 181 | rs7240004 | 18 | 46395022 | A | G | 0.501 | 0.499 | G | 0.499 | Inflammatory bowel disease | 1.0E-09 |
| 182 | rs9953366 | 18 | 46474192 | T | C | 0.340 | 0.660 | T | 0.340 | Atrial fibrillation | 2.0E-10 |
| 183 | rs2878902 | 18 | 46650437 | G | T | 0.359 | 0.641 | G | 0.359 | Height | 3.0E-10 |
| 184 | rs149615216 | 18 | 47106028 | C | T | 0.996 | 0.004 | T | 0.004 | HDL cholesterol | 3.0E-09 |
| 185 | rs117687565 | 18 | 47147524 | C | T | 0.984 | 0.016 | T | 0.016 | HDL cholesterol levels | 1.0E-19 |
| 186 | rs9964304 | 18 | 47229717 | A | C | 0.665 | 0.335 | C | 0.335 | Coronary artery disease | 1.0E-09 |
| 187 | rs77947762 | 18 | 48897509 | G | A | 0.997 | 0.003 | A | 0.003 | Apolipoprotein A1 levels | 3.0E-08 |
| 188 | rs1619249 | 18 | 49190644 | A | G | 0.678 | 0.322 | G | 0.322 | Ear morphology | 4.0E-08 |
| 189 | rs7240948 | 18 | 51864456 | C | T | 0.608 | 0.392 | T | 0.392 | Hay fever and/or eczema | 7.0E-09 |
| 190 | rs8089474 | 18 | 52391708 | G | A | 0.306 | 0.694 | G | 0.306 | Life satisfaction | 4.0E-09 |
| 192 | rs72930904 | 18 | 52607301 | C | T | 0.913 | 0.087 | T | 0.087 | Diastolic blood pressure | 3.0E-09 |
| 193 | rs12966052 | 18 | 52751639 | G | C | 0.893 | 0.107 | C | 0.107 | Depression | 4.0E-11 |
| 194 | rs34220720 | 18 | 52795084 | T | C | 0.755 | 0.245 | C | 0.245 | Neuroticism | 5.0E-11 |
| 195 | rs72930774 | 18 | 53159915 | A | G | 0.970 | 0.030 | G | 0.030 | Number of sexual partners | 2.0E-15 |
| 196 | rs812738 | 18 | 53491749 | T | C | 0.631 | 0.369 | C | 0.369 | Adventurousness | 4.0E-10 |
| 197 | rs72934570 | 18 | 53533189 | C | T | 0.971 | 0.029 | T | 0.029 | Schizophrenia | 2.0E-11 |

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 198 | rs1549319 | 18 | 53694113 | C | T | 0.202 | 0.798 | C | 0.202 | Number of sexual partners | 3.0E-08 |
| 199 | rs12963285 | 18 | 54764414 | T | C | 0.859 | 0.141 | C | 0.141 | End-stage kidney disease | 1.0E-06 |
| 200 | rs759555 | 18 | 54890563 | T | C | 0.270 | 0.730 | T | 0.270 | Diverticular disease | 1.0E-07 |
| 201 | rs4092465 | 18 | 55080437 | A | G | 0.628 | 0.372 | G | 0.372 | Alcohol consumption (drinks per week) | 4.0E-08 |
| 202 | rs41292412 | 18 | 56118358 | C | T | 0.997 | 0.003 | T | 0.003 | Apolipoprotein A1 levels | 5.0E-10 |
| 203 | rs9957145 | 18 | 56876228 | G | A | 0.820 | 0.180 | A | 0.180 | Type 2 diabetes | 8.0E-09 |
| 204 | rs17782313 | 18 | 57851097 | T | C | 0.760 | 0.240 | C | 0.240 | Obesity | 2.0E-08 |
| 205 | rs476828 | 18 | 57852587 | T | C | 0.700 | 0.300 | C | 0.300 | Coffee consumption (cups per day) | 2.0E-10 |
| 206 | rs57636386 | 18 | 58048295 | T | C | 0.771 | 0.229 | C | 0.229 | Adult body size | 3.0E-23 |
| 207 | rs17749561 | 18 | 60783211 | G | A | 0.965 | 0.035 | A | 0.035 | Follicular lymphoma | 8.0E-10 |
| 208 | rs1790946 | 18 | 67528430 | C | T | 0.533 | 0.467 | T | 0.467 | Plateletcrit | 2.0E-17 |
| 209 | rs9948784 | 18 | 72897161 | G | A | 0.986 | 0.014 | A | 0.014 | IgG glycosylation | 1.0E-06 |

Table 9-9

[Table 9-9]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 210 | rs12970291 | 18 | 73017234 | G | A | 0.974 | 0.026 | A | 0.026 | Colorectal or endometrial cancer | 5.0E-08 |
| 211 | rs10514168 | 18 | 73098949 | C | A | 0.897 | 0.103 | A | 0.103 | Motion sickness | 3.0E-09 |
| 212 | rs77094751 | 18 | 76229272 | G | A | 0.984 | 0.016 | A | 0.016 | Response to bronchodilator in chronic obstructive pulmonary disease (change in FEV1) | 6.0E-07 |
| 213 | rs78015143 | 18 | 77248691 | G | A | 0.956 | 0.044 | A | 0.044 | Heel bone mineral density | 7.0E-07 |
| 214 | rs3213409 | 19 | 17945696 | C | T | 0.996 | 0.004 | T | 0.004 | Acute megakaryoblastic leukemia,Lymphoblastic leukemia | - |
| 215 | rs1064395 | 19 | 19361735 | G | A | 0.753 | 0.247 | A | 0.247 | Bipolar disorder | 2.0E-09 |
| 216 | rs10416265 | 19 | 33605300 | A | G | 0.408 | 0.592 | A | 0.408 | Bone properties (heel) | 4.0E-12 |
| 217 | rs2316205 | 19 | 41346768 | T | C | 0.618 | 0.382 | C | 0.382 | Post bronchodilator FEV1/FVC ratio | 4.0E-07 |
| 218 | rs2287921 | 19 | 49228272 | T | C | 0.784 | 0.216 | C | 0.216 | Vitamin B levels in ischemic stroke | 9.0E-07 |
| 219 | rs35610040 | 20 | 23616469 | T | C | 0.797 | 0.203 | C | 0.203 | Plasma cystastin c levels in acute coronary syndrome | 1.0E-26 |
| 220 | rs6123471 | 20 | 36840156 | T | C | 0.611 | 0.389 | C | 0.389 | Heart rate variability traits (RMSSD) | 1.0E-08 |
| 221 | rs4239702 | 20 | 44749251 | T | C | 0.268 | 0.732 | T | 0.268 | Rheumatoid arthritis | 4.0E-14 |
| 222 | rs6015450 | 20 | 57751117 | A | G | 0.902 | 0.098 | G | 0.098 | Blood pressure | 2.0E-12 |
| 223 | rs2822388 | 21 | 15408034 | A | G | 0.915 | 0.085 | G | 0.085 | Stroke | 5.0E-07 |
| 224 | rs2822999 | 21 | 16364756 | T | G | 0.891 | 0.109 | G | 0.109 | Breast cancer | 2.0E-11 |
| 225 | rs2823048 | 21 | 16462268 | A | G | 0.867 | 0.133 | G | 0.133 | Allergic rhinitis in non-asthmatics | 8.0E-07 |

EP 4 582 539 A1

57

(continued)

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 226 | rs2823141 | 21 | 16579805 | G | A | 0.387 | 0.613 | G | 0.387 | Myopia (age of diagnosis) | 9.0E-11 |
| 227 | rs7279911 | 21 | 16797068 | G | A | 0.733 | 0.267 | A | 0.267 | Hematocrit | 3.0E-37 |
| 228 | rs1297255 | 21 | 16804367 | C | T | 0.703 | 0.297 | T | 0.297 | Lymphocyte counts | 2.0E-10 |
| 229 | rs1297265 | 21 | 16817051 | A | G | 0.627 | 0.373 | G | 0.373 | Ulcerative colitis | 7.0E-13 |
| 230 | rs2823286 | 21 | 16817938 | G | A | 0.765 | 0.235 | A | 0.235 | Ulcerative colitis | 2.0E-12 |
| 231 | rs2823357 | 21 | 16914905 | G | A | 0.506 | 0.494 | A | 0.494 | Parkinson's disease | 6.0E-07 |
| 232 | rs2823615 | 21 | 17483133 | A | T | 0.733 | 0.267 | T | 0.267 | Obesity-related traits | 5.0E-08 |
| 233 | rs2823819 | 21 | 17828291 | A | G | 0.758 | 0.242 | G | 0.242 | Attention deficit hyperactivity disorder | 7.0E-07 |
| 234 | rs62239470 | 21 | 18585224 | C | T | 0.929 | 0.071 | T | 0.071 | Diastolic blood pressure (baseline) | 1.0E-07 |
| 235 | rs1389993 | 21 | 19032949 | G | C | 0.598 | 0.402 | C | 0.402 | Feeling hurt | 4.0E-09 |
| 236 | rs2101523 | 21 | 19097889 | C | T | 0.627 | 0.373 | T | 0.373 | Word reading | 1.0E-06 |
| 237 | rs2591643 | 21 | 19349773 | T | C | 0.557 | 0.443 | C | 0.443 | Neuroblastoma or malignant cutaneous melanoma | 7.0E-07 |

Table 9-10

[Table 9-10]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 238 | rs77600076 | 21 | 19531442 | A | C | 0.988 | 0.012 | C | 0.012 | Bulimia nervosa | 1.0E-06 |
| 239 | rs13050131 | 21 | 20018118 | C | A | 0.461 | 0.539 | C | 0.461 | Educational attainment (years of education) | 4.0E-08 |
| 240 | rs1870464 | 21 | 24572913 | G | A | 0.267 | 0.733 | G | 0.267 | Systolic blood pressure (baseline) | 7.0E-07 |
| 241 | rs465401 | 21 | 28181880 | A | G | 0.383 | 0.617 | A | 0.383 | Cerebrospinal fluid neurofilament light levels | 2.0E-08 |
| 242 | rs59884963 | 21 | 30125908 | C | T | 0.790 | 0.210 | T | 0.210 | Pulse pressure | 4.0E-16 |
| 243 | rs73354869 | 21 | 30567285 | A | C | 0.666 | 0.334 | C | 0.334 | Myocardial infarction | 8.0E-08 |
| 244 | rs686364 | 21 | 31587793 | A | G | 0.581 | 0.419 | G | 0.419 | Urate levels | 1.0E-17 |
| 245 | rs1544 | 21 | 33085585 | G | A | 0.762 | 0.238 | A | 0.238 | Educational attainment (years of education) | 2.0E-09 |
| 246 | rs2834018 | 21 | 34314478 | G | A | 0.705 | 0.295 | A | 0.295 | Life satisfaction | 1.0E-06 |
| 247 | rs4817505 | 21 | 34343828 | T | C | 0.726 | 0.274 | C | 0.274 | Caffeine consumption from tea | 4.0E-11 |
| 248 | rs4817543 | 21 | 34555503 | C | T | 0.832 | 0.168 | T | 0.168 | Type 2 diabetes | 6.0E-07 |
| 249 | rs9808753 | 21 | 34787312 | A | G | 0.728 | 0.272 | G | 0.272 | Multiple sclerosis | 1.0E-07 |
| 250 | rs12482821 | 21 | 35560715 | T | G | 0.990 | 0.010 | G | 0.010 | Heel bone mineral density | 7.0E-13 |
| 251 | rs9978142 | 21 | 35652239 | A | T | 0.901 | 0.099 | T | 0.099 | Pulmonary function | 3.0E-08 |
| 252 | rs7279974 | 21 | 35666961 | C | G | 0.158 | 0.842 | C | 0.158 | Coronary artery disease | 1.0E-10 |
| 253 | rs2834442 | 21 | 35690786 | T | A | 0.349 | 0.651 | T | 0.349 | Height | 5.0E-12 |
| 254 | rs1805128 | 21 | 35821680 | C | T | 0.996 | 0.004 | T | 0.004 | QT dynamics during exer-cise | 1.0E-07 |

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 255 | rs1805127 | 21 | 35821821 | T | C | 0.326 | 0.674 | T | 0.326 | Long QT syndrome,Jervell and Lange-Nielsen syndrome,Romano-Ward syndrome,Hearing loss | - |
| 256 | rs11700925 | 21 | 36237656 | C | T | 0.829 | 0.171 | T | 0.171 | Eosinophil counts | 7.0E-09 |
| 257 | rs35068491 | 21 | 36238307 | T | C | 0.887 | 0.113 | C | 0.113 | Male-pattern baldness | 5.0E-26 |
| 258 | rs7281587 | 21 | 36565278 | G | A | 0.475 | 0.525 | G | 0.475 | IgG N-glycosylation phenotypes (multivariate analysis) | 1.0E-13 |
| 259 | rs2834963 | 21 | 36921259 | C | T | 0.507 | 0.493 | T | 0.493 | Liver enzyme levels (alkaline phosphatase) | 2.0E-15 |
| 262 | rs56016578 | 21 | 38041975 | C | T | 0.996 | 0.004 | T | 0.004 | Testicular cancer | 4.0E-07 |
| 263 | rs720470 | 21 | 38933888 | T | C | 0.664 | 0.336 | C | 0.336 | Stroke | 6.0E-09 |
| 264 | rs8130408 | 21 | 39237138 | A | Y | 0.184 | 0.816 | A | 0.184 | Body size at age 10 | 2.0E-08 |
| 265 | rs9976326 | 21 | 39776485 | A | T | 0.775 | 0.225 | T | 0.225 | B-cell acute lymphoblastic leukaemia | 1.0E-08 |
| 266 | rs2836613 | 21 | 40047176 | G | A | 0.247 | 0.753 | G | 0.247 | Heel bone mineral density | 8.0E-08 |
| 267 | rs2836618 | 21 | 40048295 | G | A | 0.716 | 0.284 | A | 0.284 | Osteoarthritis (hip) | 3.0E-11 |
| 268 | rs11702843 | 21 | 40123740 | G | A | 0.868 | 0.132 | A | 0.132 | Body mass index | 2.0E-09 |

Table 9-11

[Table 9-11]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 269 | rs9980072 | 21 | 40338817 | A | G | 0.330 | 0.670 | A | 0.330 | Fractures | 3.0E-13 |
| 270 | rs2836883 | 21 | 40466744 | G | A | 0.782 | 0.218 | A | 0.218 | Takayasu arteritis | 8.0E-07 |
| 271 | rs378108 | 21 | 40469520 | A | G | 0.597 | 0.403 | G | 0.403 | Ankylosing spondylitis | 2.0E-11 |
| 272 | rs2837554 | 21 | 41667378 | A | G | 0.251 | 0.749 | A | 0.251 | Mild to moderate chronic kidney disease | 3.0E-07 |
| 273 | rs9974899 | 21 | 42006441 | C | T | 0.704 | 0.296 | T | 0.296 | Educational attainment (years of education) | 1.0E-08 |
| 274 | rs45430 | 21 | 42746081 | C | T | 0.482 | 0.518 | C | 0.482 | Melanoma | 3.0E-08 |
| 275 | rs2776348 | 21 | 43086944 | A | C | 0.497 | 0.503 | A | 0.497 | Keratinocyte cancer (MTAG) | 2.0E-19 |
| 276 | rs2776353 | 21 | 43089017 | A | T | 0.578 | 0.422 | T | 0.422 | Basal cell carcinoma | 2.0E-12 |
| 277 | rs2851391 | 21 | 44487404 | T | C | 0.385 | 0.615 | T | 0.385 | Blood metabolite levels | 1.0E-11 |
| 278 | rs148593140 | 21 | 45282969 | G | C | 0.989 | 0.011 | C | 0.011 | Blood protein levels | 5.0E-09 |
| 279 | rs59998884 | 21 | 45618114 | T | C | 0.334 | 0.666 | T | 0.334 | Inflammatory bowel disease | 1.0E-09 |
| 280 | rs6518350 | 21 | 45621817 | A | G | 0.841 | 0.159 | G | 0.159 | Type 1 diabetes | 1.0E-07 |
| 281 | rs58911644 | 21 | 45629121 | A | T | 0.833 | 0.167 | T | 0.167 | Celiac disease | 6.0E-07 |
| 282 | rs4819388 | 21 | 45647421 | T | C | 0.282 | 0.718 | T | 0.282 | Celiac disease | 2.0E-09 |
| 283 | rs116969723 | 21 | 45876716 | G | A | 0.977 | 0.023 | A | 0.023 | Intracranial aneurysm | 4.0E-15 |
| 284 | rs2838923 | 21 | 46846944 | A | G | 0.508 | 0.492 | G | 0.492 | Hippocampal atrophy | 8.0E-07 |
| 285 | rs12482088 | 21 | 46901973 | A | C | 0.792 | 0.208 | C | 0.208 | Blood protein levels | 1.0E-19 |
| 286 | rs116274237 | 21 | 46910352 | C | G | 0.787 | 0.213 | G | 0.213 | Platelet distribution width | 1.0E-11 |

(continued)

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 287 | rs2150458 | 21 | 47377296 | G | A | 0.377 | 0.623 | G | 0.377 | Spherical equivalent or myopia (age of diagnosis) | 1.0E-14 |
| 288 | rs186825689 | 21 | 48006053 | A | C | 0.994 | 0.006 | C | 0.006 | Cannabis dependence symptom count | 8.0E-08 |
| 289 | rs2530664 | 22 | 30038152 | A | C | 0.785 | 0.215 | C | 0.215 | Neurofibromatosis | - |
| 290 | rs1012068 | 22 | 32265903 | T | G | 0.613 | 0.387 | G | 0.387 | Liver cancer | 1.0E-13 |
| 291 | rs3818120 | 22 | 41523770 | G | A | 0.910 | 0.090 | A | 0.090 | Colorectal carcinoma | - |
| 292 | rs738409 | 22 | 44324727 | C | G | 0.738 | 0.262 | G | 0.262 | Type 2 diabetes | 8.0E-07 |
| 293 | rs5979785 | X | 12971524 | C | T | 0.426 | 0.574 | C | 0.426 | Celiac disease | 6.0E-08 |
| 294 | rs5955543 | X | 17698397 | A | G | 0.748 | 0.252 | G | 0.252 | Wilms tumor | 1.0E-09 |
| 295 | rs1378559 | X | 21380266 | T | C | 0.879 | 0.121 | C | 0.121 | Schizophrenia | 2.0E-12 |
| 296 | rs73456411 | X | 29737404 | G | T | 0.928 | 0.072 | T | 0.072 | Vitiligo | 7.0E-10 |
| 297 | rs6631478 | X | 32092380 | T | C | 0.653 | 0.347 | C | 0.347 | Ankle-brachial index | 7.0E-08 |
| 298 | rs56156506 | X | 37999652 | A | T | 0.624 | 0.376 | T | 0.376 | Anorexia nervosa | 1.0E-06 |

Table 9-12

[Table 9-12]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 299 | rs35318931 | X | 38009121 | G | A | 0.979 | 0.021 | A | 0.021 | Sex hormone-binding globulin levels | 4.0E-09 |
| 300 | rs17145638 | X | 39885630 | T | C | 0.904 | 0.096 | C | 0.096 | Pit-and-Fissure caries | 2.0E-07 |
| 301 | rs142513793 | X | 47906480 | C | T | 0.978 | 0.022 | T | 0.022 | Tuberculosis | 2.0E-07 |
| 302 | rs5952553 | X | 49392721 | C | T | 0.595 | 0.405 | T | 0.405 | Vitiligo | 1.0E-09 |
| 303 | rs147570790 | X | 49837621 | G | T | 0.971 | 0.029 | T | 0.029 | Red blood cell count | 1.0E-10 |
| 304 | rs1934179 | X | 50182184 | G | A | 0.572 | 0.428 | A | 0.428 | Hypospadias | 3.0E-21 |
| 305 | rs4554617 | X | 50203402 | A | C | 0.606 | 0.394 | C | 0.394 | Hypospadias | 1.0E-93 |
| 306 | rs45479691 | X | 55047741 | G | A | 0.984 | 0.016 | A | 0.016 | Mean corpuscular hemoglobin | 4.0E-20 |
| 307 | rs185597083 | X | 55133845 | C | A | 0.993 | 0.007 | A | 0.007 | Male-pattern baldness | 1.0E-11 |
| 308 | rs149290585 | X | 55704361 | T | C | 0.993 | 0.007 | C | 0.007 | Mean corpuscular hemoglobin | 1.0E-09 |
| 309 | rs7064929 | X | 64367019 | G | A | 0.738 | 0.262 | A | 0.262 | Erectile dysfunction and prostate cancer treatment | 7.0E-07 |
| 310 | rs73225807 | X | 66312646 | T | A | 0.967 | 0.033 | A | 0.033 | Reticulocyte count | 4.0E-09 |
| 311 | rs147676232 | X | 67932642 | C | T | 0.993 | 0.007 | T | 0.007 | Bioavailable testosterone levels | 1.0E-08 |
| 312 | rs4360450 | X | 70146398 | A | G | 0.188 | 0.812 | A | 0.188 | Uterine fibroids | 2.0E-18 |
| 313 | rs6625947 | X | 71354911 | C | T | 0.413 | 0.587 | C | 0.413 | Plateletcrit | 1.0E-09 |
| 314 | rs201634149 | X | 75127170 | C | CT | 0.986 | 0.014 | T | 0.014 | Mean reticulocyte volume | 3.0E-09 |
| 315 | rs5912838 | X | 78497118 | A | C | 0.546 | 0.454 | C | 0.454 | Thyrotoxic hypokalemic periodic paralysis and Graves disease | 6.0E-08 |

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 316 | rs7886910 | X | 78630857 | T | C | 0.013 | 0.987 | T | 0.013 | Birth weight | 3.0E-12 |
| 317 | rs5922945 | X | 83523015 | C | T | 0.787 | 0.213 | T | 0.213 | Dentures | 2.0E-07 |
| 318 | rs2710057 | X | 86192003 | G | T | 0.283 | 0.717 | G | 0.283 | Bone mineral density (hip) | 1.0E-06 |
| 319 | rs34360385 | X | 90407072 | C | T | 0.687 | 0.313 | T | 0.313 | Metopic nonsyndromic craniosynostosis | 6.0E-07 |
| 320 | rs2071226 | X | 100662681 | T | C | 0.977 | 0.023 | C | 0.023 | Fabry disease | - |
| 321 | rs7060620 | X | 101012848 | A | T | 0.776 | 0.224 | T | 0.224 | Chronotype | 1.0E-11 |
| 322 | rs17331777 | X | 102362908 | A | G | 0.832 | 0.168 | G | 0.168 | Educational attainment (years of education) | 4.0E-08 |
| 323 | rs1180894 | X | 102750889 | G | A | 0.897 | 0.103 | A | 0.103 | Bioavailable testosterone levels | 1.0E-09 |
| 324 | rs874 | X | 102930960 | A | G | 0.661 | 0.339 | G | 0.339 | Mean platelet volume | 4.0E-11 |
| 325 | rs12007422 | X | 109902012 | T | G | 0.649 | 0.351 | G | 0.351 | Fasting insulin | 1.0E-06 |
| 326 | rs144832584 | X | 109906103 | C | T | 0.988 | 0.012 | T | 0.012 | Low density lipoprotein cholesterol levels | 2.0E-08 |
| 327 | rs5929166 | X | 112877567 | G | A | 0.992 | 0.008 | A | 0.008 | Poor prognosis in Crohn's disease | 5.0E-09 |

Table 9-13

[Table 9-13]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 328 | rs73578101 | X | 114576974 | G | A | 0.893 | 0.107 | A | 0.107 | Mean platelet volume | 5.0E-15 |
| 329 | rs5191 | X | 115304276 | G | A | 0.988 | 0.012 | A | 0.012 | | - |
| 330 | rs7879546 | X | 115348275 | T | C | 0.481 | 0.519 | T | 0.481 | Lung disease severity in cystic fibrosis | 1.0E-09 |
| 331 | rs5952223 | X | 115386565 | C | T | 0.744 | 0.256 | T | 0.256 | Lung disease severity in cystic fibrosis | 5.0E-08 |
| 332 | rs4825885 | X | 123259530 | A | G | 0.344 | 0.656 | A | 0.344 | Mean reticulocyte volume | 4.0E-31 |
| 333 | rs723557 | X | 126653357 | T | G | 0.936 | 0.064 | G | 0.064 | Prostate cancer (survival) | 6.0E-07 |
| 334 | rs16999497 | X | 128735199 | T | C | 0.855 | 0.145 | C | 0.145 | Diarrhoea in darapladib-treated cardiovascular disease (time to event) | 6.0E-09 |
| 336 | rs59933080 | X | 131253361 | T | C | 0.874 | 0.126 | C | 0.126 | Heel bone mineral density | 3.0E-10 |
| 337 | rs12392108 | X | 131314262 | T | A | 0.638 | 0.362 | A | 0.362 | Uterine fibroids and heavy menstrual bleeding | 5.0E-07 |
| 338 | rs12863738 | X | 136032127 | C | T | 0.740 | 0.260 | T | 0.260 | Pediatric autoimmune diseases | 6.0E-08 |
| 339 | rs7067005 | X | 139314460 | C | T | 0.868 | 0.132 | T | 0.132 | Myopia (age of diagnosis) | 2.0E-22 |
| 340 | rs17002540 | X | 139946061 | C | T | 0.820 | 0.180 | T | 0.180 | Esophageal adenocarcinoma x pack-years of smoking exposure interaction | 6.0E-07 |
| 341 | rs11095909 | X | 140967165 | T | C | 0.561 | 0.439 | C | 0.439 | Type 2 diabetes | 5.0E-07 |

(continued)

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 342 | rs57403204 | X | 141078552 | A | G | 0.907 | 0.093 | G | 0.093 | High-grade serous ovarian cancer | 2.0E-07 |
| 343 | rs16994583 | X | 147554909 | G | A | 0.968 | 0.032 | A | 0.032 | Monocyte percentage of white cells | 3.0E-12 |
| 344 | rs113116706 | X | 147558205 | T | C | 0.968 | 0.032 | C | 0.032 | Basophil count | 3.0E-17 |
| 345 | rs148665432 | X | 147683026 | C | T | 0.990 | 0.010 | T | 0.010 | Basophil percentage of white cells | 7.0E-09 |
| 346 | rs13397 | X | 153248248 | G | A | 0.652 | 0.348 | A | 0.348 | Celiac disease | 3.0E-08 |
| 347 | rs3890298 | X | 153413519 | C | G | 0.726 | 0.274 | G | 0.274 | Lymphocyte counts | 1.0E-11 |
| 348 | rs17336718 | X | 153536119 | C | T | 0.952 | 0.048 | T | 0.048 | Testicular germ cell tumor | 4.0E-08 |
| 349 | rs6655282 | X | 153546556 | G | A | 0.709 | 0.291 | A | 0.291 | High light scatter reticulocyte percentage of red cells | 5.0E-26 |
| 350 | rs2664170 | X | 153945602 | G | A | 0.353 | 0.647 | G | 0.353 | Type 1 diabetes | 8.0E-09 |
| 351 | rs114209171 | X | 154278797 | T | C | 0.758 | 0.242 | C | 0.242 | Thrombosis | 7.0E-13 |
| 352 | rs7051718 | X | 154332656 | T | C | 0.770 | 0.230 | C | 0.230 | Venous thromboembolism | 4.0E-12 |
| 353 | rs306890 | X | 154987147 | T | C | 0.706 | 0.294 | C | 0.294 | Triglycerides | 8.0E-12 |
| 354 | rs3093479 | X | 155230627 | A | G | 0.794 | 0.206 | G | 0.206 | Eosinophil counts | 3.0E-11 |
| 355 | rs9785971 | Y | 6753511 | A | G | 0.173 | 0.827 | A | 0.173 | Autism | 7.0E-08 |
| 356 | rs1865680 | Y | 6868118 | A | G | 0.266 | 0.734 | A | 0.266 | Autism | 2.0E-08 |

Table 9-14

[Table 9-14]

| No. | dbSNP (rs No.) | Chr. No. | position | reference allele | alternative allele | reference allele freq. | alternative allele freq. | minor allele | minor allele freq. | DISEASE/TRAIT | P-VALUE |
|---|---|---|---|---|---|---|---|---|---|---|---|
| 357 | rs2032624 | Y | 15026424 | A | C | 0.758 | 0.242 | C | 0.242 | Autism | 2.0E-08 |
| 358 | rs2032658 | Y | 15581983 | G | A | 0.266 | 0.734 | G | 0.266 | Autism | 3.0E-09 |
| 359 | rs2115848 | Y | 16804852 | G | A | 0.000 | 1.000 | | 0.000 | Pursuit maintenance gain | 1.0E-06 |
| 360 | rs2032631 | Y | 21867787 | A | G | 0.303 | 0.697 | A | 0.303 | Autism | 2.0E-08 |
| 361 | rs9786153 | Y | 22739367 | C | T | 0.173 | 0.827 | C | 0.173 | Autism | 3.0E-09 |

**Claims**

1. A method for genetic analysis of a set of targets of analysis comprising multiple single nucleotide polymorphism loci, wherein:

   the set of targets of analysis meets at least two of the following conditions (i) to (iii):

   (i) The ratio of the number of single nucleotide polymorphism loci, which is known to have a phenotype to the total number of single nucleotide polymorphism loci in the targets is 10% or more;
   (ii) The ratio of the number of single nucleotide polymorphism loci, among the total number of single nucleotide polymorphism loci in the set of targets of analysis, where the minor allele frequency is 1% to 50% and which are separated from each other by a distance of 20 kb or more on a chromosome, is 10% or more;
   (iii) The ratio of the number of single nucleotide polymorphism loci present on chromosome 13, chromosome 18, chromosome 21, chromosome X, and chromosome Y to the total number of single nucleotide polymorphism loci in the set of targets of analysis is 10% or more;

   comprising a genotyping step, wherein:

   the genotyping step includes analyzing a child's nucleic acid sample containing a nucleic acid with the genetic information of a child, and a father's nucleic acid sample containing a nucleic acid with the genetic information of a father and/or a mother's nucleic acid sample containing a nucleic acid with the genetic information of a mother,
   to acquire data including:

   - allele sequence information , which is multiple single nucleotide polymorphism loci in the set of targets of analysis in the nucleic acids of each sample; and
   - numerical values reflecting the genomic region containing these loci and/or the relative or absolute abundance of the alleles;

   further comprising executing at least two steps selected from the following: a Phenotype Analysis Step, a Parentage Testing Step, and a calculation step for evaluating chromosomal abnormalities, based on the data obtained from a single analysis,
   wherein when the Phenotype Analysis Step is included, the set of targets of analysis meets condition (i), when the Parentage Testing Step is included, the set of targets of analysis meets condition (ii), and when the calculation step for evaluating chromosomal abnormalities is included, the set of targets of analysis meets condition (iii).
   <Step for Phenotype Analysis>
   A step of analyzing, based on the genotyping results of single nucleotide polymorphism loci, which is known to be associated with the phenotype, obtained in the genotyping step, the likelihood of expression of the phenotype associated with the minor allele at the single nucleotide polymorphism loci, in at least the child sample,
   (excluding the diagnosis of a disease in a human).
   <Parentage Testing Step>
   A step of verifying the existence of a parent-child relationship, comprising:

   - determining a Paternity Index (PI) for each of the plurality of single nucleotide polymorphism loci based on the result of the genotyping step and multiplying the PIs to obtain a Combined Paternity Index (CPI), or
   - determining a Maternity Index (MI) for each of the plurality of single nucleotide polymorphism loci based on the result of the genotyping step and multiplying the MIs to obtain a Combined Maternity Index (CMI),

   wherein PI or MI is calculated as follows for the following cases:

   - the child is a fetus before birth and paternity testing is performed,
   - the child is after birth and paternity testing is performed, or
   - the child is after birth and maternity testing is performed.

   Case where the child is a fetus before birth and paternity testing is performed:

The child's nucleic acid sample is a cell-free nucleic acid sample collected from the pregnant mother with the child.

- When genotyping of the father and the fetus is performed by analyzing two types of samples, the cell-free nucleic acid sample and the father's nucleic acid sample, PI is determined based on Table 1 or Table 2.
- When genotyping of the father, the mother, and the fetus is performed by analyzing three types of samples, the cell-free nucleic acid sample, the father's nucleic acid sample, and the mother's nucleic acid sample, PI is determined based on Table 3 or Table 4.

Case where the child is after birth and paternity testing is performed:

- When genotyping of the father and the child is performed by analyzing two types of samples, the child's nucleic acid sample and the father's nucleic acid sample, PI is determined based on Table 5.
- When genotyping of the father, the mother, and the child is performed by analyzing three types of samples, the child's nucleic acid sample, the father's nucleic acid sample, and the mother's nucleic acid sample, PI is determined based on Table 6.

Case where the child is after birth and maternity testing is performed:

- When genotyping of the mother and the child is performed by analyzing two types of samples, the child's nucleic acid sample and the mother's nucleic acid sample, MI is determined based on Table 7.
- When genotyping of the father, the mother, and the child is performed by analyzing three types of samples, the child's nucleic acid sample, the father's nucleic acid sample, and the mother's nucleic acid sample, MI is determined based on Table 8.

Table 1

| CASE | Genotype | | Paternity Index |
|------|----------|----|-----------------|
| | alleged father | fetus | PI |
| (I) | AA | AA | $\dfrac{1-k_0}{(1-k_0)a+k_0 b}$ |
| (II) | AA | AB | $\dfrac{k_b}{k_b a+(1-k_b)b}$ |
| (III) | BB | AA | $\dfrac{k_0}{(1-k_0)a+k_0 b}$ |
| (IV) | BB | AB | $\dfrac{1-k_b}{k_b a+(1-k_b)b}$ |
| (V) | AB | AA | $\dfrac{1}{2(1-k_0)a+2k_0 b}$ |
| (VI) | AB | AB | $\dfrac{1}{2k_b a+2(1-k_b)b}$ |

Table 2

| CASE | Genotype | | Paternity Index |
|------|----------|----|-----------------|
| | alleged father | fetus | PI |
| (I) | AA | AA | 1/a |
| (II) | AA | AB | kb |

(continued)

| CASE | Genotype | | Paternity Index |
|---|---|---|---|
| | alleged father | fetus | PI |
| (III) | BB | AA | k0 |
| (IV) | BB | AB | 1/b |
| (V) | AB | AA | 0.5/a |
| (VI) | AB | AB | 0.5/b |

Table 3

| CASE | Genotype | | | Paternity Index |
|---|---|---|---|---|
| | mother | alleged father | fetus | PI |
| (I) | AA | AA | AA | $\dfrac{1-k_0}{(1-k_0)a + k_0 b}$ |
| (II) | AA | AA | AB | $\dfrac{k_b}{k_b a + (1-k_b)b}$ |
| (III) | AA | BB | AA | $\dfrac{k_0}{(1-k_0)a + k_0 b}$ |
| (IV) | AA | BB | AB | $\dfrac{1-k_b}{k_b a + (1-k_b)b}$ |
| (V) | AA | AB | AA | $\dfrac{1}{2(1-k_0)a + 2k_0 b}$ |
| (VI) | AA | AB | AB | $\dfrac{1}{2k_b a + 2(1-k_b)b}$ |

Table 4

| CASE | Genotype | | | Paternity Index |
|---|---|---|---|---|
| | mother | alleged father | fetus | PI |
| (I) | AA | AA | AA | 1/a |
| (II) | AA | AA | AB | kb |
| (III) | AA | BB | AA | k0 |
| (IV) | AA | BB | AB | 1/b |
| (V) | AA | AB | AA | 0.5/a |
| (VI) | AA | AB | AB | 0.5/b |

Table 5

| CASE | Genotype | | Paternity Index |
|---|---|---|---|
| | alleged father | child | PI |
| (I) | AA | AA | 1/a |
| (II) | AA | AB | 0.5/a |
| (III) | BB | AA | $\mu/\beta$ |

(continued)

| CASE | Genotype | | Paternity Index |
|------|----------|--|-----------------|
| | alleged father | child | PI |
| (IV) | AB | AA | 0.5/a |
| (V) | AB | AB | (a+b) 4ab |

Table 6

| CASE | Genotype | | | Paternity Index |
|------|----------|--|--|-----------------|
| | mother | alleged father | child | PI |
| (I) | AA | AA | AA | 1/a |
| (II) | AA | AA | AB | $\mu/\beta$ |
| (III) | AA | BB | AA | $\mu/\alpha$ |
| (IV) | AA | BB | AB | 1/b |
| (V) | AA | AB | AA | 0.5/a |
| (VI) | AA | AB | AB | 1/ (a+b) |

Table 7

| CASE | Genotype | | Paternity Index |
|------|----------|--|-----------------|
| | alleged mather | child | PI |
| (I) | AA | AA | 1/a |
| (II) | AA | AB | 0.5/a |
| (III) | BB | AA | $\mu/\beta$ |
| (IV) | AB | AA | 0.5/a |
| (V) | AB | AB | (a+b) 4ab |

Table 8

| CASE | Genotype | | | Paternity Index |
|------|----------|--|--|-----------------|
| | alleged mother | father | child | PI |
| (I) | AA | AA | AA | 1/a |
| (II) | AA | AA | AB | $\mu/\beta$ |
| (III) | AA | BB | AA | $\mu/\alpha$ |
| (IV) | AA | BB | AB | 1/b |
| (V) | AA | AB | AA | 0.5/a |
| (VI) | AA | AB | AB | 1/ (a+b) |

In Tables 1 to 4, the allele with relatively high signal intensity in the analysis of the cell-free nucleic acid sample is referred to as A, and the allele with relatively low signal intensity is referred to as B.

In Tables 1 and 2, k0 is a false negative rate, kb is a false positive rate, a is the frequency of A, and b is the frequency of B.

In Tables 5 to 8, $\mu$ is the mutation rate, a and b are the occurrence frequencies of allele A and allele B, respectively, $\alpha$ is the average exclusion power of allele A, and $\beta$ is the average exclusion power of allele B.

<Calculation Step for Evaluating Chromosomal Numerical Abnormalities>

A step of calculating a Z score or NCV (Normalized Chromosome Value) as an index to evaluate the copy number variation of chromosome 13, chromosome 18, chromosome 21, chromosome X, and chromosome Y in at least the child, based on the numerical values contained in the data,

wherein the Z score (Z chrN) for chromosome N is calculated according to the following equation (1), and the NCV (NCV chrN by chrM) for chromosome N relative to reference chromosome M is calculated according to the following equation (2).

$$Z_{chrN}$$
$$= \frac{proportion\ of\ chr\ N - mean\ proportion\ of\ chr\ N\ in\ reference\ disomic\ population}{SD\ of\ proportion\ of\ chr\ N\ in\ reference\ disomic\ population}$$

...equation (1)

(In equation (1), N is 13, 18, 21, X, or Y,

"proportion of chr N" is the ratio of the numerical value reflecting the abundance of chromosome N to the total numerical value reflecting the abundance of all chromosomes except chromosomes 13, 18, 21, X, and Y, when analyzing the child's nucleic acid sample.

"mean proportion of chr N in reference disomic population" is the mean ratio of the numerical value reflecting the abundance of chromosome N to the total numerical value reflecting the abundance of all chromosomes except chromosomes 13, 18, 21, X, and Y, when analyzing multiple standard samples with a normal disomic karyotype.

"SD of proportion of chr N in reference disomic population" is the standard deviation of the ratio of the numerical value reflecting the abundance of chromosome N to the total numerical value reflecting the abundance of all chromosomes except chromosomes 13, 18, 21, X, and Y, when analyzing multiple standard samples with a normal disomic karyotype.)

$$NCV_{chrN\ by\ chr\ M}$$
$$= \frac{Normalized\ proportion\ of\ chr\ N\ by\ chr\ M - mean\ normalized\ proportion\ of\ chr\ N\ in\ reference\ disomic\ population}{SD\ of\ normalized\ proportion\ of\ chr\ N\ in\ reference\ disomic\ population}$$

...equation (2)

(In equation (2),

"Normalized proportion of chr N by chr M" is the ratio of the numerical value reflecting the abundance of chromosome N relative to the numerical value reflecting the abundance of chromosome M in the child's nucleic acid sample.

"mean normalized proportion of chr N in reference disomic population" is the mean ratio of the numerical value reflecting the abundance of chromosome N relative to the numerical value reflecting the abundance of chromosome M when analyzing multiple standard samples with a normal disomic karyotype.

"SD of normalized proportion of chr N in reference disomic population" is the standard deviation of the ratio of the numerical value reflecting the abundance of chromosome N relative to the numerical value reflecting the abundance of chromosome M when analyzing multiple standard samples with a normal disomic karyotype.)

2. The genetic analysis method according to claim 1, comprising all of the Parentage Testing Step, the calculation step for evaluating chromosomal numerical abnormalities, and the Phenotype Analysis Step.

3. The genetic analysis method according to claim 1 or 2, wherein the set of targets of analysis satisfies all the conditions set forth in (i) to (iii), and the ratio of the number of single nucleotide polymorphism loci that are known to have a phenotype, have a minor allele frequency of 1% to 50%, are separated from each other by a distance of 20 kb or more on a chromosome, and present on chromosome 13, chromosome 18, chromosome 21, chromosome X, and chromosome Y, to the total number of single nucleotide polymorphism loci constituting the set of targets of analysis is 10% or more.

4. The genetic analysis method according to any one of claims 1 to 3, wherein the child is a fetus in the mother's womb, and the child's nucleic acid sample is a cell-free nucleic acid sample collected from the mother.

**5.** The genetic analysis method according to any one of claims 1 to 3, wherein the child is postnatal.

Fig.1

CH+F

Fig.2

M+CH+F

## INTERNATIONAL SEARCH REPORT

| International application No. |
|---|
| **PCT/JP2023/019597** |

| A. | CLASSIFICATION OF SUBJECT MATTER |
|---|---|

*C12N 15/09*(2006.01)i; *C12Q 1/68*(2018.01)i; *C12Q 1/6827*(2018.01)i; *C12Q 1/6851*(2018.01)i; *C12Q 1/6869*(2018.01)i
FI: C12Q1/68 100Z; C12Q1/6851 Z; C12Q1/6869 Z; C12N15/09 Z; C12Q1/6827 Z

According to International Patent Classification (IPC) or to both national classification and IPC

| B. | FIELDS SEARCHED |
|---|---|

Minimum documentation searched (classification system followed by classification symbols)

C12N15/09; C12Q1/68; C12Q1/6827; C12Q1/6851; C12Q1/6869

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2009-519703 A (APPLERA CORP.) 21 May 2009 (2009-05-21) <br> paragraphs [0079], [0216], [0263], [0270], tables 1-4, 6-15 | 1-5 |
| A | JP 2014-502845 A (NATERA, INC.) 06 February 2014 (2014-02-06) <br> claims 1-3, paragraphs [0024], [0050], [0053], [0087] | 1-5 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 July 2023** | **08 August 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT
### Information on patent family members

International application No.

**PCT/JP2023/019597**

| Patent document cited in search report | | | Publication date (day/month/year) | Patent family member(s) | Publication date (day/month/year) |
|---|---|---|---|---|---|
| JP | 2009-519703 | A | 21 May 2009 | US 2005/0272054 A1<br>paragraphs [0114], [0269], [0319], [0326], tables 1-4, 6-15 | |
| JP | 2014-502845 | A | 06 February 2014 | US 2012/0122701 A1<br>claims 1-3, paragraphs [0024], [0058], [0061], [0094] | |

Form PCT/ISA/210 (patent family annex) (January 2015)

**REFERENCES CITED IN THE DESCRIPTION**

**Patent documents cited in the description**

- JP 2014502845 A
- JP 2022519159 A
- JP 2019153332 A
- WO 7121440 A

**Non-patent literature cited in the description**

- *Nutrients*, 21 February 2020, vol. 12 (2), 566
- *Prenatal Diagnosis*, 04 March 2020, vol. 40, 497-506
- *PLoS One*, 15 September 2016, vol. 11 (9), e0159385
- *PLoS One*, 12 September 2019, vol. 14 (9), e0222535
- *PLoS One*, 12 April 2019, vol. 14 (4), e0215368
- *Clin. Chem.*, July 2011, vol. 57 (7), 1042-9